(19) Europäisches Patentamt
European Patent Office
Office européen des brevets

(11) Veröffentlichungsnummer: **0 168 812**
**A1**

(12) **EUROPÄISCHE PATENTANMELDUNG**

(21) Anmeldenummer: **85108885.6**

(22) Anmeldetag: **16.07.85**

(51) Int. Cl.⁴: **C 07 D 401/04**, A 61 K 31/47

(30) Priorität: **18.07.84 US 631971**

(43) Veröffentlichungstag der Anmeldung: **22.01.86**
**Patentblatt 86/4**

(84) Benannte Vertragsstaaten: **AT BE CH DE FR GB IT LI LU NL SE**

(71) Anmelder: **F. HOFFMANN-LA ROCHE & CO. Aktiengesellschaft, CH-4002 Basel (CH)**

(72) Erfinder: **Field, George Francis, 33 McKinley Avenue, West Caldwell, N.J. (US)**
Erfinder: **Zally, William Joseph, 24 Milton Street, Cresskill, N.J. (US)**

(74) Vertreter: **Lederer, Franz, Dr. et al, Patentanwälte Dr. Lederer Franz Meyer-Roxlau Reiner F. Lucile-Grahn-Strasse 22, D-8000 München 80 (DE)**

(54) **4-Amino-2-phenylchinoline, Verfahren und Zwischenprodukte zu deren Herstellung sowie diese enthaltende Arzneimittel.**

(57) Die Verbindungen der Formel

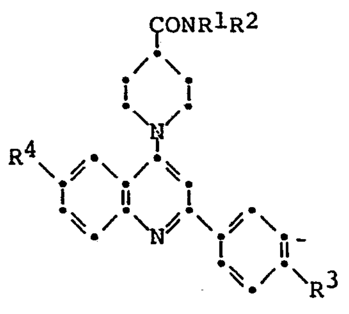

I

worin $R^1$ und $R^2$ unabhängig voneinander Wasserstoff oder nieder Alkyl und $R^3$ und $R^4$ unabhängig voneinander Wasserstoff, Halogen oder nieder Alkyl bedeuten, mit der Auflage, dass $R^3$ und $R^4$ nicht gleichzeitig Wasserstoff bedeuten, sind nützlich als Antikonvulsiva und Anxiolytica. Sie können hergestellt werden durch Umsetzung entsprechender 4-Chlor-2-phenylchinoline mit entsprechenden 4-Piperidincarboxamiden oder durch Aminierung von entsprechenden freien Säuren, Säurechloriden oder Estern.

EP 0 168 812 A1

ACTORUM AG

0 168 812

F. HOFFMANN-LA ROCHE & CO. Aktiengesellschaft, Basel/Schweiz

16. Juli 1985

RAN 4008/335



4-Amino-2-phenylchinoline, Verfahren und Zwischenprodukte zu deren Herstellung sowie diese enthaltende Arzneimittel

Die vorliegende Erfindung betrifft 4-Amino-2-phenylchinoline der allgemeinen Formel

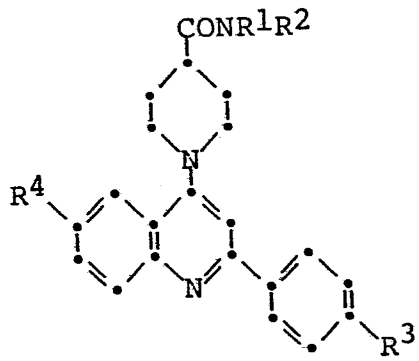

I

worin $R^1$ und $R^2$ unabhängig voneinander Wasserstoff oder nieder Alkyl und $R^3$ und $R^4$ unabhängig voneinander Wasserstoff, Halogen oder nieder Alkyl bedeuten, mit der Auflage, dass $R^3$ und $R^4$ nicht gleichzeitig Wasserstoff bedeuten.

Der in der vorliegenden Beschreibung verwendete Ausdruck "nieder Alkyl" bezieht sich auf geradkettige und verzweigte Kohlenwasserstoffreste mit 1 bis 8, vorzugsweise 1 bis 4, Kohlenstoffatomen, wie beispielsweise Methyl, Aethyl,

Kbr/28.6.1985

Propyl, Isopropyl, tert. Butyl und dergleichen. Der in dieser Beschreibung verwendete Ausdruck "nieder Alkoxy" bezieht sich auf Alkyläthergruppen, in denen die Alkylgruppe die obige Bedeutung besitzt. Der Ausdruck "Halogen" umfasst die Halogenatome Fluor, Chlor, Brom und Jod.

Im Rahmen der vorliegenden Erfindung besonders bevorzugte niedere Alkylgruppen sind Methyl und Aethyl.

$R^3$ bedeutet vorzugsweise Chlor, Brom, Methyl oder Aethyl.

Besonders bevorzugte Verbindungen der Formel I sind:

1-[2-(4-Chlorphenyl)-4-chinolinyl]-N-äthyl-4-piperidincarboxamid,

1-[2-(4-Chlorphenyl)-6-fluor-4-chinolinyl]-4-piperidincarboxamid,

1-[2-(4-Chlorphenyl)-4-chinolinyl]-N-methyl-4-piperidincarboxamid und

1-[2-(4-Chlorphenyl)-4-chinolinyl]-4-piperidincarboxamid.

Die 4-Amino-2-phenylchinoline der Formel I können erfindungsgemäss hergestellt werden, indem man

(a) ein 4-Halo-2-phenylchinolin der allgemeinen Formel

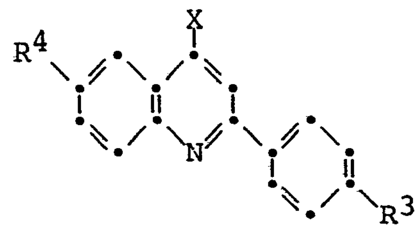

II

worin $R^3$ und $R^4$ die oben angegebene Bedeutung besitzen und X ein Halogenatom bedeutet,

mit einem Isonipecotamid der allgemeinen Formel

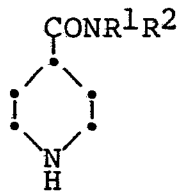

III

worin $R^1$ und $R^2$ die oben angegebene Bedeutung besitzen,

umsetzt, oder

(b) eine Verbindung der allgemeinen Formel

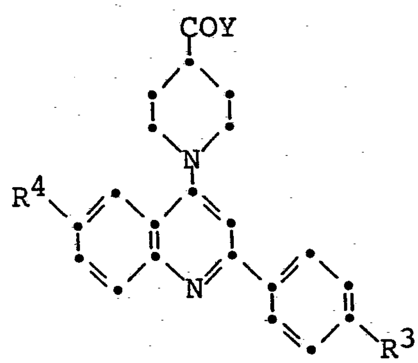

IV

worin $R^3$ und $R^4$ die oben angegebene Bedeutung besitzen und Y Halogen, Hydroxy oder nieder Alkoxy bedeutet, mit einem Amin der allgemeinen Formel

$$NHR^1R^2 \qquad V$$

worin $R^1$ und $R^2$ die oben angegebene Bedeutung besitzen,

umsetzt.

Die Umsetzung eines 4-Halo-2-phenylchinolins der Formel II mit einem Isonipecotamid der Formel III, welches entweder eine bekannte Verbindung ist oder leicht nach bekannten Methoden hergestellt werden kann, gemäss Verfahrensvariante (a) kann nach an sich bekannten Methoden durchgeführt werden, entweder in Gegenwart oder Abwesenheit eines Lösungsmittels. Geeignete Lösungsmittel, falls überhaupt Lösungsmittel verwendet werden, sind niedere Alkanole, Phenole oder andere Lösungsmittel, die sich für nucleophile Austauschreaktionen eignen, wie Dimethylformamid, Dimethylsulfoxid und Hexamethylphosphoramid. Die Umsetzung kann bei einer Temperatur im Bereich zwischen etwa 70° und 200°C, vorzugsweise bei etwa 150°C, durchgeführt werden. Besonders bevorzugte Lösungsmittel sind Phenol und Aethanol. Falls kein Lösungsmittel verwendet wird, kann das Chinolin der Formel II mit einem Ueberschuss des Isonipecotamids umgesetzt werden. Diese Umsetzung wird bei einer Temperatur im Bereich zwischen etwa 100° und 180°C durchgeführt.

Die Umsetzung eines Säurehalogenids der Formel IV, d.h. einer Verbindung der Formel IV, worin Y Halogen bedeutet, mit einem Amin der Formel V, welches entweder eine bekannte Verbindung ist oder leicht nach bekannten Methoden hergestellt werden kann, gemäss Verfahrensvariante (b) kann in inerten Lösungsmitteln, wie chlorierten Kohlenwasserstoffen, Aethern oder Kohlenwasserstoffen, beispielsweise Methylenchlorid, Tetrahydrofuran oder Toluol, bei einer Temperatur

im Bereich zwischen etwa 0° und 100°C, vorzugsweise bei etwa 20°C, durchgeführt werden.

Standardmethoden zur Umwandlung von Estern oder Säuren in Amide können verwendet werden, um Verbindungen der Formel IV, worin Y Hydroxy oder nieder Alkoxy bedeutet, in Verbindungen der Formel I überzuführen. Beispielsweise liefert die Behandlung eines Gemisches einer Säure der Formel IV mit einem Amin der Formel V, wie Aethylamin, in einem inerten Lösungsmittel mit einem Dehydrierungsmittel, wie Dicyclohexylcarbodiimid, ein Amid der Formel I.

Die 4-Halo-2-phenylchinoline der Formel II, von denen viele noch neu sind, können nach verschiedenen Methoden hergestellt werden.

So können beispielsweise Verbindungen der Formel II, worin X Chlor bedeutet, hergestellt werden, wie im nachfolgenden Reaktionsschema 1 veranschaulicht, in welchem $R^3$ und $R^4$ die obige Bedeutung besitzen.

Reaktionsschema 1

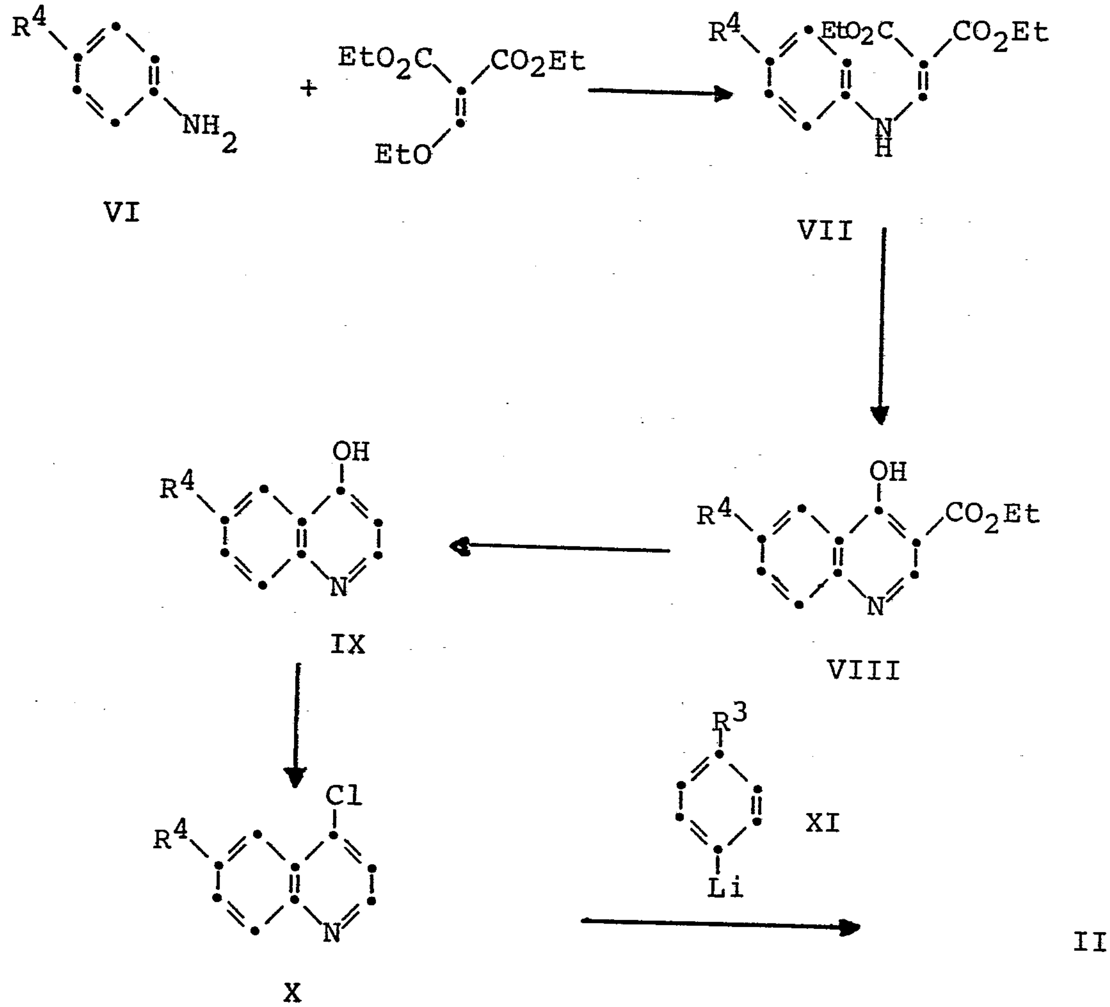

VI VII VIII IX X XI II

Die Kondensation eines substituierten Anilins der Formel VI, welches entweder eine bekannte Verbindung ist oder leicht nach bekannten Verfahren hergestellt werden kann, mit Aethyläthoxymethylenmalonat erfolgt in an sich bekannter Weise durch Erhitzen eines Gemisches der Komponenten, entweder in Abwesenheit oder Gegenwart eines Lösungsmittels, wie Diphenyläther oder Toluol, auf eine Temperatur von ungefähr 120°C. Die erhaltene Verbindung der Formel VII wird danach nach bekannten Methoden cyclisiert, beispielsweise durch Erhitzen auf eine Temperatur von ungefährt 200° bis 250°C, in Gegenwart eines inerten organischen Lösungsmittels, wie Diphenyläther.

Das erhaltene Chinolin der Formel VIII wird danach nach bekannten Methoden decarboxyliert, indem man zuerst den Ester mit einer Base, wie wässriges Natriumhydroxyd, bei einer Temperatur von beispielsweise 100°C hydrolysiert und danach die erhaltene Säure entweder in einem inerten organischen Lösungsmittel wie Diphenyläther oder ohne Lösungsmittel auf eine Temperatur von ungefährt 250°C erhitzt.

Das erhaltene 4-Hydroxychinolin der Formel IX wird danach nach bekannten Methoden mit einem Chlorierungsmittel wie Phosphoroxychlorid zum 4-Chlorchinolin der Formel X umgesetzt.

Das 4-Chorchinolin der Formel X wird schliesslich nach bekannten Methoden mit einer Phenyllithiumverbindung der Formel XI zum erwünschten 4-Chlor-2-phenylchinolin der Formel II umgesetzt. Diese Umsetzung kann beispielsweise in einem inerten Lösungsmittel, wie Diäthyläther oder Tetrahydrofuran, bei einer Temperatur im Bereich zwischen etwa -80° und 20°C unter Inertgasatmosphäre durchgeführt werden.

Das untenstehende Reaktionsschema 2 illustriert eine Alternativroute zur Herstellung der erwünschten Ausgangsstoffe der Formel II. Wiederum besitzen $R^3$ und $R^4$ die oben angegebene Bedeutung.

Reaktionsschema 2

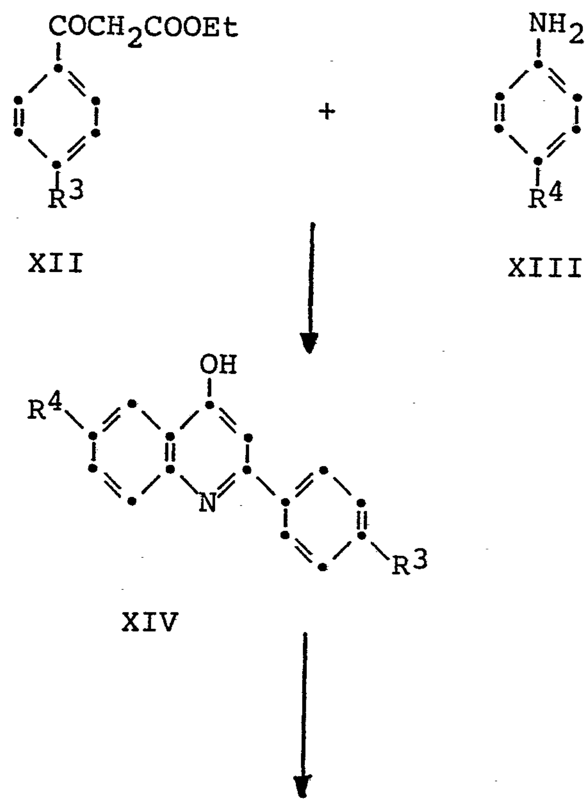

XII

XIII

XIV

II

Sowohl der Benzoylessigsäureäthylester der Formel XII als auch das substituierte Anilin der Formel XIII sind bekannt oder können leicht nach bekannten Methoden hergestellt werden.

Die Umsetzung der Verbindungen der Formeln XII und XIII erfolgt nach an sich bekannten Methoden, wobei zunächst die beiden Verbindungen in Gegenwart eines inerten organischen Lösungsmittels, wie Toluol, und einer milden Säure, wie p- Toluolsulfonsäure, unter Rückfluss kondensiert werden. Das Produkt wird unter vermindertem Druck eingeengt und danach durch Erhitzen auf eine Temperatur zwischen etwa 200°

und 260°C in einem inerten organischen Lösungsmittel, wie Diphenyläther, unter Bildung des 4-Hydroxy-2-phenylchinolins der Formel XIV cyclisiert.

Die Umsetzung eines Chinolins der Formel XIV mit einem Halogenierungsmittel, wie Phosphoroxychlorid oder Phosphortribromid, bei Rückflusstemperatur und gegebenenfalls in einem inerten organischen Lösungsmittel, wie Diphenyläther, liefert das erwünschte Ausgangsmaterial der Formel II.

Eine weitere Methode zur Herstellung der Ausgangsstoffe der Formel II ist im nachfolgenden Reaktionsschema 3 illustriert, in welchem $R^3$ und $R^4$ die oben angegebene Bedeutung besitzen und $R^5$ nieder Alkyl bedeutet.

Reaktionsschema 3

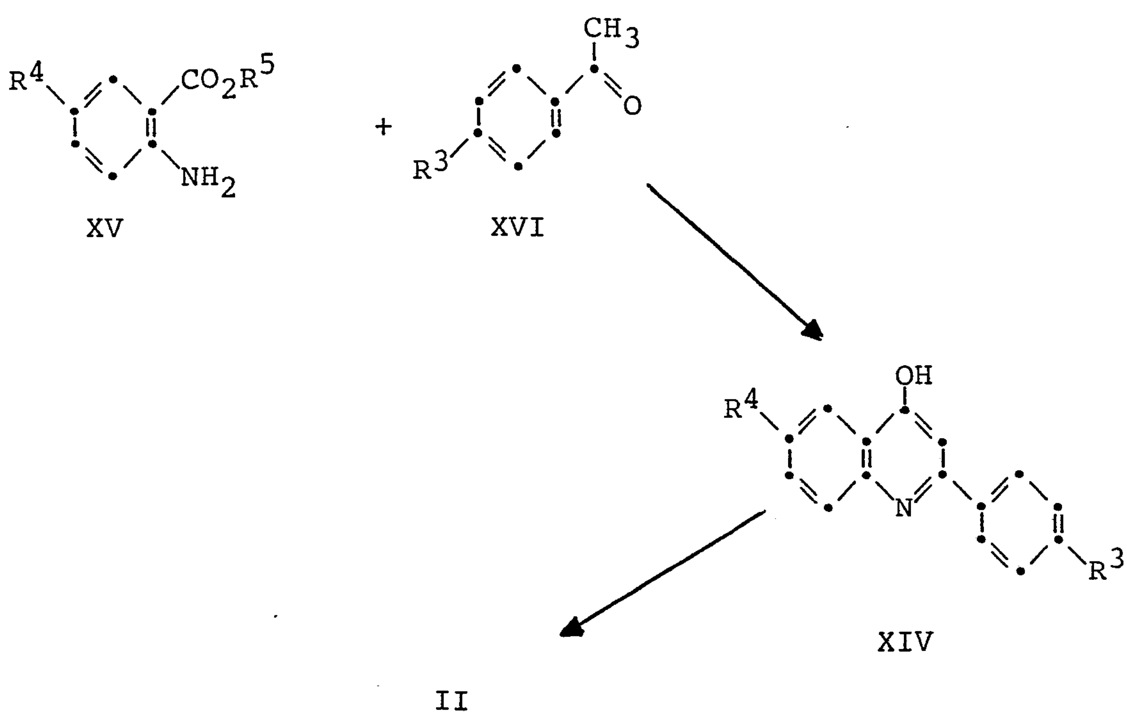

XV + XVI → XIV → II

Sowohl der Anthranilsäureester der Formel XV als auch das substituierte Acetophenon der Formel XVI sind bekannt oder können leicht nach bekannten Methoden hergestellt werden.

Nach diesem Syntheseweg erfolgt zunächst eine Kondensation der Verbindungen der Formeln XV und XVI in Gegenwart einer Lewissäure, wie Aluminiumchlorid, in einem hochsiedenden Lösungsmittel, wie Diphenyläther, bei der Rückflusstemperatur unter Bildung einer Verbindung der Formel XIV. Die weitere Umsetzung dieser Verbindung zum Ausgangsmaterial der Formel II erfolgt wie im Reaktionsschema 2 beschrieben.

Eine weitere Methode zur Herstellung von Verbindungen der Formel II ist im untenstehenden Reaktionsschema 4 veranschaulicht, in welchem $R^3$, $R^4$ und X die oben angegebene Bedeutung besitzen.

<u>Reaktionsschema 4</u>

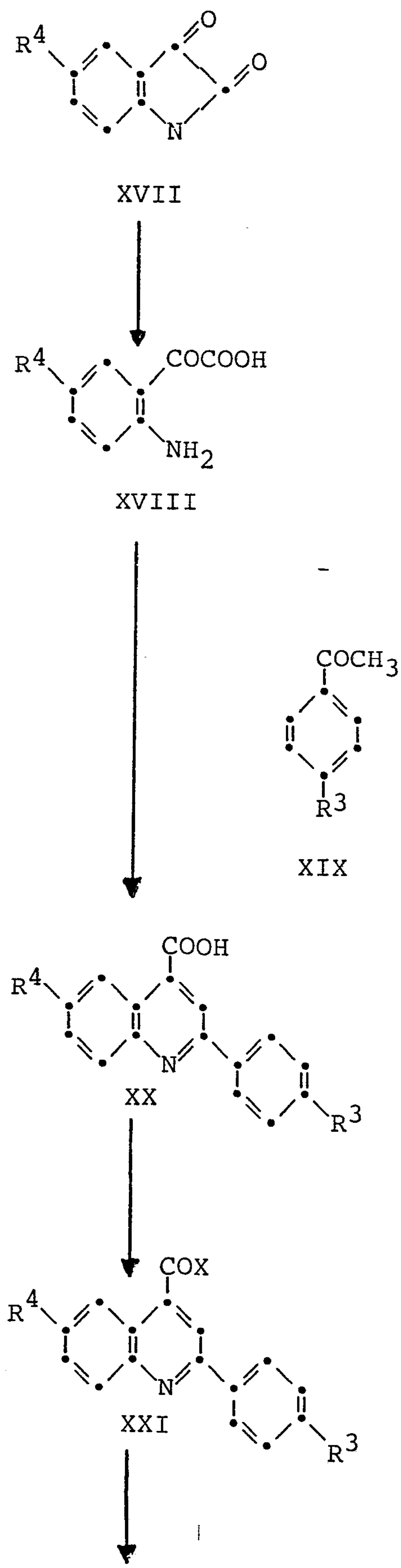

XVII

XVIII

XIX

XX

XXI

II

Sowohl das substituierte Isatin der Formel XVII als auch das substituierte Acetophenon der Formel XIX sind entweder bekannt oder können leicht nach bekannten Methoden hergestellt werden.

Die Ueberführung eines Isatins der Formel XVII in eine Verbindung der Formel XVIII erfolgt nach bekannten Methoden und umfasst die Behandlung des substituierten Isatins mit einer Base, wie beispielsweise Natriumhydroxyd in Aethanol.

Die Kondensation einer Verbindung der Formel XVIII mit einem Acetophenon der Formel XIX erfolgt ebenfalls nach konventionellen Methoden durch Erhitzen der beiden Reaktanten zur Rückflusstemperatur in einem Lösungsmittel, wie Aethanol, in Gegenwart einer Base, wie Natriumhydroxyd.

Die Ueberführung der Säure der Formel XX in das Säurehalogenid der Formel XXI erfolgt nach konventionellen Methoden durch Umsetzen der Säure mit einem Halogenierungsmittel, wie Thionylchlorid oder Phosphortribromid, unter Rückfluss in einem inerten organischen Lösungsmittel, wie Toluol.

Das Säurehalogenid der Formel XXI wird schliesslich durch Decarbonylierung unter Verwendung eines Standardreagens, wie beispielsweise eines Rhodiumkatalysators, bei erhöhter Temperatur in das erwünschte 4-Halo-2-phenylchinolin übergeführt.

Die Ausgangsstoffe der Formel IV sind neu und bilden ebenfalls Gegenstand vorliegender Erfindung. Sie können hergestellt werden, indem man zunächst eine Verbindung der Formel II mit einem Isonipecotinsäurederivat der allgemeinen

Formel

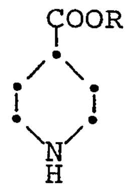

XXII

worin R nieder Alkyl bedeutet, umsetzt, wobei man eine Verbindung der Formel IV erhält, worin Y nieder Alkoxy bedeutet, d.h. eine Verbindung der allgemeinen Formel

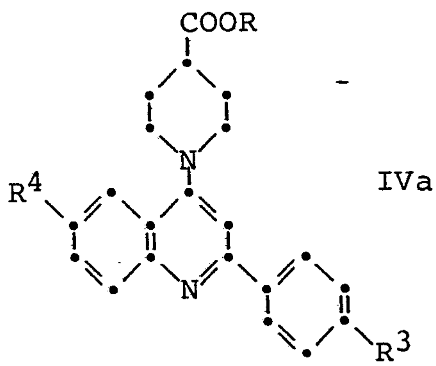

IVa

worin R, $R^3$ und $R^4$ die oben angegebene Bedeutung besitzen.

Erwünschtenfalls kann der erhaltene Ester danach zur entsprechenden Verbindung der Formel IV, worin Y Hydroxy bedeutet, d.h. zu einer Verbindung der allgemeinen Formel

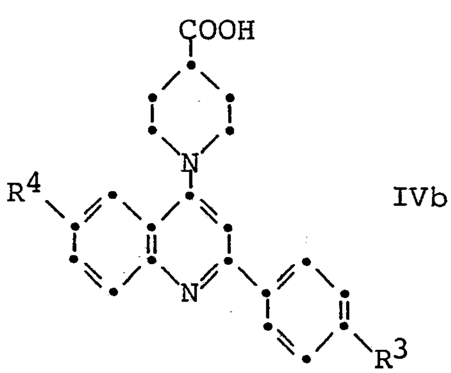

IVb

worin $R^3$ und $R^4$ die oben angegebene Bedeutung besitzen,

hydrolysiert werden.

Erwünschtenfalls kann die Carbonsäure danach mit einem Halogenierungsmittel unter Bildung einer Verbindung der Formel IV, worin Y Halogen bedeutet, d.h. einer Verbindung der allgemeinen Formel

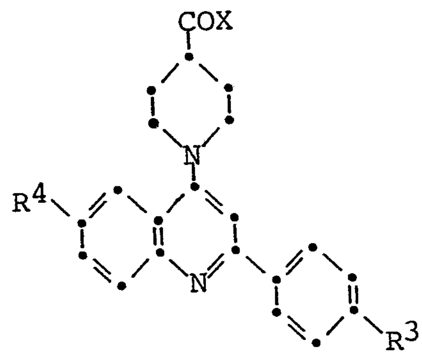

IVc

worin $R^3$, $R^4$ und X die oben angegebene Bedeutung besitzen,

umgesezt werden.

Es wurde festgestellt, dass die Verbindungen der Formel I wertvolle Anxiolytika darstellen. In den nachfolgend beschriebenen Testverfahren wurden die Verbindungen der Formel I auf ihre Antimetrazolaktivität und $^3$H-Diazepam--Bindungsaktivität getestet. Die 24 Stunden Akut-Toxizität ($LD_{50}$ in mg/kg) von jeder der getesteten Verbindungen wurde ebenfalls nach Standardmethoden bestimmt. Die Resultate dieser Versuche sind in der nachfolgenden Tabelle 1 zusammengestellt.

Intravenöser Antipentetrazol-Test (Ptz.)

45 bis 54 Tage alte männliche CF-1 Mäuse, welche während einer Woche zusammen waren und für 24 Stunden kein Futter erhalten hatten, wurden in diesem Test eingesetzt. Die in 5% Akazienöl dispergierte Testverbindung wurde oral verabreicht, zunächst 3 Mäusen bei 1/10 Dosis der Lethaldosis, die vorgängig bestimmt worden war. Eine Stunde später wurde das Pentetrazol intravenös verabreicht (70 mg/kg; konvulsive Dosis 100 mg/kg) und die Versuchstiere 30 Sekunden lang auf einen Schutz gegen Konvulsionen beobachtet. Falls die Verbindung oral aktiv war, wurden 8 Versuchstiere pro Dosis eingesetzt. Die Dosis (in mg/kg), bei der 50% der Versuchstiere vor Krampfanfällen geschützt sind, wird als $ED_{50}$ bezeichnet. Berechnungen: Die $ED_{50}$-Werte und die 95%-Vertrauensbereiche werden mit einem Computerprogramm nach der Methode von D.J. Finney berechnet ("Probit Analysis", Cambridge University Press, Cambridge, England, 1971).

Gemäss dieser Methode wurden die Aktivitäten für Standardverbindungen wie folgt bestimmt:

| Verbindung | $ED_{50}$ (95% Vertrauensbereiche) mg/kg |
|---|---|
| Chlordiazepoxyd | 3.9 (2.3 - 5.9) |
| Diazepam | 1.0 (0.69 - 1.6) |
| Natrium-Phenobarbital | 19.0 (9.7 - 29) |

## $^3$H-Diazepam-Bindungstest ($^3$H-Dz.)

Corticalfragmente von Rattenhirn wurden präpariert und das Bindungsverfahren nach der Methode von Mohler und Okada (Life Sciences, 20, 2102, 1977) durchgeführt, ausgenommen dass anstelle von Krebs-Puffer Tris-Puffer verwendet wurde. Die Testverbindungen wurden dreimal geprüft. Die Radioaktivität wurde mittels Flüssigkeits-Scintillationszählung bestimmt. Die Resultate werden als $IC_{50}$ angegeben, was der Konzentration der Testverbindung (in nMol/Liter) entspricht, welche notwendig ist, $^{3}H$-Diazepambindung zu 50% zu hemmen. Nach dieser Methode wurden die Aktivitäten von Standardverbindungen wie folgt bestimmt:

| Verbindung | $IC_{50}$ |
|---|---|
| Diazepam | 5.0 nMol/l |
| Flunitrazepam | 1.8 nMol/l |
| Flurazepam | 15.6 nMol/l |

Tabelle I

| Verbindung der Formel I | Ptz. $ED_{50}$ (mg/kg) | $^{3}H$-Dz. $IC_{50}$ (nMol/l) | $LD_{50}$ (mg/kg) |
|---|---|---|---|
| 1-[2-(4-Chlorphenyl)-4-chinolinyl]-N-äthyl-4-piperidincarboxamid | 26 | 9.0 | >1000 |
| 1-[2-(4-Chlorphenyl-4-chinolinyl]-N,N-diäthyl-4-piperidincarboxamid | - | 500 | - |
| 1-[2-(4-Chlorphenyl)-6-fluor-4-chinolinyl]-4-piperidincarboxamid | 15 | 36.0 | >1000 |
| 1-[2-(4-Chlorphenyl)-4-chinolinyl]-N-methyl-4-piperidincarboxamid | 9 | 19.5 | >1000 |

| | | | |
|---|---|---|---|
| 1-[2-(4-Chlorphenyl)-4-chinolinyl]-4-piperidincarboxamid | 24 | 29.0 | >1000 |
| 1-[2-(4-Fluorphenyl)-4-chinolinyl]-4-piperidincarboxamid | >100 | 1.1 | >1000 |
| 1-[6-Fluor-2-phenyl-4-chinolinyl]-4-piperidincarboxamid | 120 | 3.6 | >1000 |
| N-Aethyl-1-[6-fluor-2-phenyl-4-chinolinyl]-4-piperidincarboxamid | 58 | 2.8 | >1000 |
| 1-[6-Fluor-2-phenyl-4-chinolinyl]-N-propyl-4-piperidincarboxamid | >100 | 13.0 | >1000 |
| 1-[6-Fluor-2-(4-fluorphenyl)-4-chinolinyl]-4-piperidincarboxamid | 5 | 1.0 | >1000 |
| 1-[6-Fluor-2-(4-fluorphenyl)-4-chinolinyl]-N-methyl-4-piperidincarboxamid | 13 | 1.8 | >1000 |
| N-Aethyl-1-[6-fluor-2-(4-fluorphenyl)-4-chinolinyl]-4-piperidincarboxamid | 22 | 3.0 | >1000 |
| 1-[2-(4-Methylphenyl)-4-chinolinyl]-4-piperidincarboxamid | 37 | 1.7 | >1000 |

| | | | |
|---|---|---|---|
| 1-[2-(4-Bromphenyl)-4-chinolinyl]-4-piperidincarboxamid | 41 | 35.0 | >1000 |
| 1-[6-Fluor-2-(4-methylphenyl)-4-chinolinyl]-4-piperidincarboxamid | 88 | 5.4 | >1000 |
| 1-[6-Chlor-2-(4-methylphenyl)-4-chinolinyl]-4-piperidincarboxamid | >100 | 60.0 | >1000 |
| 1-[2-(4-Chlorphenyl)-6-fluor-4-chinolinyl]-N-methyl-4-piperidincarboxamid | >300 | 70.0 | >1000 |
| 1-[6-Chlor-2-(4-fluorphenyl)-4-quinolinyl]-4-piperidincarboxamid | 29 | 28.0 | >1000 |
| 1-[6-Chlor-2-(4-fluorphenyl)-4-chinolinyl]-N-methyl-4-piperidincarboxamid | 37 | 10.5 | >1000 |
| 1-[6-Chlor-2-(4-chlorphenyl)-4-chinolinyl]-4-piperidincarboxamid | - | 205.0 | - |
| 1-[6-Chlor-2-phenyl-4-chinolinyl]-4-piperidincarboxamid | 50 | 96.0 | >1000 |

| | | | |
|---|---|---|---|
| 1-[6-Methyl-2-phenyl-4-chinolinyl]-4-piperidin-carboxamid | 90 | 28.0 | >1000 |
| 1-[2-(4-Fluorphenyl)-6-methyl-4-chinolinyl]-4-piperidincarboxamid | >100 | 34.0 | >1000 |
| 1-[6-Methyl-2-(4-methylphenyl)-4-chinolinyl]-4-piperidincarboxamid | - | 290.0 | - |
| 1-[2-(4-Chlorphenyl)-6-methyl-4-chinolinyl]-4-piperidincarboxamid | - | 460.0 | - |
| 1-[2-(4-Chlorphenyl)-4-chinolinyl]-N,N-dimethyl-4-piperidincarboxamid | 24 | 80.0 | >1000 |

">" gibt an, dass die Verbindungen oberhalb des angegebenen Wertes nicht getestet wurden.

Die Verbindungen der Formel I sind wertvoll bei der Behandlung von Krampfanfällen oder Angstzuständen, wenn in geeigneten Dosierungseinheiten verabreicht.

Eine geeignete Dosierung für eine Verbindung der Formel I liegt im Bereich von ungefähr 10 bis 500 mg pro Tag, vorzugsweise im Bereich von etwa 25 bis 200 mg, in einer oder mehreren Dosen. Es ist jedoch darauf hinzuweisen, dass die Verabreichung von Patient zu Patient variiert, und dass die Dosis für einen bestimmten Patienten von der Beurteilung durch den Arzt abhängt, welcher als Kriterien zur Bestimmung einer geeigneten Dosis die Körpergrösse und die Verfassung des Patienten sowie dessen Reaktion auf den Wirkstoff in

Betracht ziehen wird.

Die Verbindungen der Formel I können als Medikamente in Form pharmazeutischer Präparate verabreicht werden, welche diese zusammen mit einem kompatiblen pharmazeutischen Trägermaterial enthalten. Ein solches Trägermaterial kann ein inertes organisches oder anorganisches Trägermaterial sein, welches für die parenterale oder enterale Verabreichung geeignet ist, beispielsweise für die orale Verabreichung. Beispiele solcher Trägermaterialien sind Wasser, Gelatine, Talk, Stärke, Magnesiumstearat, Gummi arabicum, pflanzliche Oele, Polyalkylenglykole, Vaseline und dergleichen. Die pharmazeutischen Präparate können nach konventionellen Methoden hergestellt werden, und die fertigen Dosierungsformen können feste Dosierungsformen sein, beispielsweise Tabletten, Dragées, Suppositorien, Kapseln und dergleichen, oder flüssige Dosierungsformen, beispielsweise Lösungen, Suspensionen, Emulsionen und dergleichen. Die pharmazeutischen Präparate können konventionellen pharmazeutischen Operationen unterworfen werden, wie Sterilisation. Im weiteren können die pharmazeutischen Präparate übliche Hilfsstoffe enthalten, wie Konservierungsmittel, Stabilisierungsmittel, Emulgiermittel, Mittel zur Verbesserung des Geschmacks, Netzmittel, Puffer, Salze zur Veränderung des osmotischen Drucks und dergleichen.

Die folgenden Beispiele veranschaulichen die Erfindung weiter; alle Temperaturen sind in Celsiusgraden angegeben.

## Beispiel 1

Herstellung des Ausgangsmaterials 4-(N-Aethylcarbamoyl)piperidin

(a) Methylisonicotinat

Eine Suspension von 100 g Isonicotinsäure (0,812 Mol) in 250 ml Methanol wird gerührt und auf 10° gekühlt. Zu dieser Mischung werden tropfenweise 125 ml Schwefelsäure innerhalb von 15 Minuten gegeben, wobei die Temperatur unter 20° gehalten wird. Man lässt das Reaktionsgemisch auf Raumtemperatur aufwärmen und erhitzt für 4 1/2 Stunden zum Rückfluss. Nach Stehenlassen über Nacht wird das Reaktionsgemisch auf 1 kg Eis gegossen und mit 235 g Natriumcarbonat alkalisch gestellt. Der Festkörper wird abfiltriert, mit Wasser und Aether gewaschen und verworfen. Das Filtrat wird mit 3 x 300 ml Aether extrahiert. Die vereinigten organischen Extrakte werden mit Wasser und gesättigter Kochsalzlösung gewaschen, über Natriumsulfat getrocknet und unter vermindertem Druck eingeengt, wobei man 80 g (65%) Rohprodukt als helles Oel erhält.

(b) N-Aethylisonicotinamid

Man kühlt ein Gemisch von 200 ml Aethanol und 20 ml Wasser in einem Eisbad, sättigt dieses während einer halben Stunde mit Aethylamin und gibt danach unter Kühlen 80 g rohes Methylisonicotinat zu. Während 10 Minuten wird danach nochmals Aethylamin in das Reaktionsgemisch eingeleitet, und dieses über Nacht bei Raumtemperatur stehen gelassen. Das Reaktionsgemisch wird danach unter vermindertem Druck eingedampft, wobei man 82.82 g eines hellen Oels erhält, welches beim Abkühlen halbfest wird.

(c) 4-(N-Aethylcarbamoyl)piperidin

Eine Lösung von 82.8 g (0.551 Mol) N-Aethylisonicotinamid in 600 ml Aethanol wird über 5.0 g Platinoxyd bei 60° und 1.425 MPa während 5 Stunden hydriert. Man lässt danach das Reaktionsgemisch über Nacht abkühlen und filtriert den Katalysator ab. Das Filtrat wird unter vermindertem Druck eingedampft. Der Rückstand wird aus Aether kristallisiert, wobei man 62.09 g Rohprodukt erhält.

## Beispiel 2

In analoger Weise wie in Beispiel 1 beschrieben, wurden die folgenden Ausgangsstoffe hergestellt:

a) 4-(N-Methylcarbamoyl)piperidin

b) 4-(N-Propylcarbamoyl)piperidin.

## Beispiel 3

Herstellung von 1-[2-(4-Chlorphenyl)-4-chinolinyl]-N--äthyl-4-piperidincarboxamid

(a) 4-Chlor-2-(4-chlorphenyl)chinolin

Zu einer auf -25° gekühlten Lösung von 15.3 g (80 mMol) 4-Bromchlorbenzol in 100 ml Aether werden 60 ml einer 1.6M Lösung von Butyllithium in Hexan in 3 Portionen gegeben, wobei man die Temperatur unterhalb von -20° hält. Man lässt das Gemisch nachher während 15 Minuten auf -5° erwärmen. Danach wird es wiederum auf -20° abgekühlt, und eine Lösung von 11.45 g (70 mMol) 4-Chlorchinolin in 40 ml Aether wird zuge- geben, wobei man die Temperatur bei -20° hält. Eine gelbe Suspension wird gebildet. Diese lässt man während 15 Minuten auf 0° erwärmen und rührt dann während 20 weiteren Minuten bei 0 bis 10°. Danach werden zum Reaktionsgemisch 20 ml Wasser und 20 g Jod gegeben. Nach 20minütigem Rühren werden 40 ml einer 3N Natriumhydroxydlösung zugegeben und weitere 15 Minuten gerührt. Das Gemisch wird dann auf 10° abgekühlt, und der Festkörper abgetrennt, wobei man 17.65 g des Produkts erhält, Schmelzpunkt 119 - 122°.

(b) 1-[2-(4-Chlorphenyl)-4-chinolinyl] -4-piperidincarbonsäureäthylester

Ein Gemisch von 10 g (36.4 mMol) 4-Chlor-2-(4-chlorphenyl)chinolin und 20 ml Aethylisonipecotat wird gerührt und während 2 Stunden auf 160° bis 170° erhitzt. Danach wird das Gemisch abgekühlt und mit 200 ml Wasser und 5 ml Aethanol verdünnt. Der gebildete Festkörper wird abgetrennt und aus Aethanol umkristallisiert, wobei man 5.5 g des obigen Esters erhält, Schmelzpunkt 124 - 126°.

(c) 1-[2-(4-Chlorphenyl)-4-chinolinyl] -4-piperidincarbonsäure

Ein Gemisch von 1.5 g (3.8 mMol) 1-[2-(4-Chlorphenyl)-4-chinolinyl] -4-piperidincarbonsäureäthylester, 2.7 ml 3N wässrige Natriumhydroxydlösung und 50 ml Aethanol wird auf einem Dampfbad während 30 Minuten erhitzt. Das Gemisch wird danach gekühlt, mit Essigsäure angesäuert und mit Wasser verdünnt, wobei man 1.4 g des Produkts erhält, Schmelzpunkt 245 - 247°.

(d) 1-[2-(4-Chlorphenyl)-4-chinolinyl] -4-piperidincarbonylchlorid

Ein Gemisch von 8.0 g der oben hergestellten Säure, 24 ml Thionylchlorid und 400 ml Methylenchdlorid wird gerührt und während 1 Stunde zum Rückfluss erhitzt. Dieses Reaktionsgemisch wird nachher unter vermindertem Druck eingeengt, und der Rückstand mit Aether abgetrennt, wobei man 8.0 g des Säurechlorids als gelben Festkörper erhält.

(e) 1-[2-(4-Chlorphenyl)-4-chinolinyl] -N-äthyl-4-piperidincarboxamid

Zu einer bei ungefähr 10° mit Aethylamin gesättigten Lösung von 100 ml Methylenchlorid werden 2.0 g 1-[2-(4-Chlorphenyl-4-chinolinyl] -4-piperidincarbonyl-

chlorid unter Rühren gegeben. Das Reaktionsgemisch wird danach während 1 Stunde bei Raumtemperatur gerührt. Danach wird es mit 3 x 500 ml Wasser gewaschen, über Natriumsulfat getrocknet und unter vermindertem Druck eingedampft. Der Rückstand wird aus Aether umkristallisiert, wobei man 1.3 g weisse Nadeln erhält, Schmelzpunkt 231 - 233°.

## Beispiel 4

Alternative Herstellung des Zwischenprodukts 4-Chlor -2-(4-chlorphenyl)chinolin

(a) 2-(4-Chlorphenyl)-4-chinolinol

Zu einer gerührten Mischung von 16.52 g (0.1 Mol) o-Aminobenzoesäureäthylester, 15.46 g (0.1 Mol) p-Chloracetophenon und 250 ml Diphenyläther werden vorsichtig 18.67 g Aluminiumchlorid gegeben. Dieses Gemisch wird während 2 Stunden zum Rückfluss erhitzt, und danach lässt man auf 60° abkühlen. Der Festkörper wird abgetrennt und zweimal mit 100 ml Toluol gewaschen. Dieser Festkörper wird danach zweimal mit einem Gemisch von 500 ml 6N Salzsäure und 50 ml Aceton angerieben. Jedesmal wird auf diese Weise ein Festkörper abgetrennt und mit 100 ml 6N Salzsäure, 3 x 100 ml Wasser und 2 x 50 ml Aceton gewaschen. Auf diese Weise erhält man 22.34 g des obengenannten Produkts, Schmelzpunkt 327 - 330°.

(b) 4-Chlor-2-(4-chlorphenyl)chinolin

Ein Gemisch von 21.6 g 2-(4-Chlorphenyl)-4-chinolinol und 46 ml Phosphoroxychlorid wird während 45 Minuten zum Rückfluss erhitzt, auf Raumtemperatur abgekühlt und vorsichtig auf 750 ml Eiswasser gegossen. Das Gemisch wird mit 146 ml einer 50%igen Natriumhydroxydlösung neutralisiert und mit 600 ml Methylenchlorid in drei Teilen extrahiert. Die Extrakte werden vereinigt, durch 15 g Florisil filtriert und

unter vermindertem Druck eingeengt, wobei man 19.35 g des obengenannten Produkts erhält.

Beispiel 5

Herstellung von 1-[2-(4-Chlorphenyl)-4-chinolinyl] -N,N-diäthyl-4-piperidincarboxamid

In analoger Weise wie in Beispiel 3 (e), jedoch unter Verwendung von Diäthylamin anstelle von Aethylamin, wird die obengenannte Verbindung hergestellt, Schmelzpunkt 155 - 157° (Zers.) aus Aethanol.

Beispiel 6

Verfahren zur Herstellung von 1-[2-(4-Chlorphenyl) -6-fluor-4-chinolinyl]-4-piperidincarboxamid

(a) 2-(4-Chlorphenyl)-6-fluor-4-hydroxychinolin

Ein Gemisch von 140 g (0.617 Mol) Aethyl 4-chlorbenzoylacetat, 500 ml Toluol, 59 ml (0.621 Mol) 4-Fluoranilin und 3 g p-Toluonsulfonsäure wird gerührt und während 4 Stunden zum Rückfluss erhitzt. Das Reaktionsgemisch wird danach unter vermindertem Druck eingeengt, wobei man 187 g eines Rückstands erhält. Der in 75 ml Diphenyläther gelöste Rückstand wird unter Rühren auf einmal zu 500 ml auf 245° erhitztem Diphenyläther gegeben. Man lässt das Reaktionsgemisch auf 40° abkühlen und verdünnt danach mit Hexan. Der Festkörper wird abgetrennt und mit Methylenchlorid gewaschen, wobei man 56 g des Rohprodukts erhält, Schmelzpunkt >350°.

(b) 4-Chlor-2-(4-chlorphenyl)-6-fluorchinolin

Ein Gemisch von 25 g (91 mMol) von 2-(4-Chlorphenyl-6--fluor-4-hydroxychinolin und 50 ml Phosphoroxychlorid wird unter Rühren 2 Stunden zum Rückfluss erhitzt. Das Gemisch

wird danach abgekühlt, auf Eis gegossen und mit 500 ml Methylenchlorid verdünnt. Dieses Gemisch wird danach mit 3N Natriumhydroxydlösung alkalisch gestellt. Die organische Phase wird abgetrennt, und die wässrige Phase mit 2 x 200 ml Methylenchlorid extrahiert. Die organischen Extrakte werden vereinigt, mit Wasser und gesättigter Kochsalzlösung gewaschen, über Natriumsulfat getrocknet und unter vermindertem Druck eingedampft. Der erhaltene Rückstand wird mit Hexan gewaschen, wobei man 22.2 g des Produkts als bräunlichen Festkörper erhält, Schmelzpunkt 169 - 172°.

(c) 1-[2-(4-Chlorphenyl)-6-fluor -4-chinolinyl]-4-piperidincarboxamid

Ein Gemisch von 4.4 g (15 mMol) 4-Chlor-2-(4-chlorphenyl) -6-fluorchinolin, 3.9 g (30 mMol) Isonipecotamid und 6 g Phenol wird unter Rühren während 2 Stunden in einem Oelbad auf 165° bis 180° erhitzt. Das Gemisch wird dann abgekühlt und mit 100 ml Wasser verdünnt. Beim Anreiben tritt Kristallisation ein. Der Festkörper wird abgetrennt und mit Aether gewaschen, wobei man 5.2 g des Rohprodukts erhält, Schmelzpunkt 258 - 260° (Zers.). Umkristallisation aus Pyridin/Wasser liefert weissliche Nadeln, Schmelzpunkt 259 - 261°.

## Beispiel 7

Herstellung von 1-[2-(4-Chlorphenyl)-4-chinolinyl]-N-methyl-4-piperidincarboxamid

Zu einer Lösung von 100 ml Methylenchlorid, welche bei ungefähr 10° mit Methylamin gesättigt worden ist, werden unter Rühren 2.0 g des in Beispiel 3 (d) beschriebenen Säurechlorids gegeben. Das Reaktionsgemisch wird eine Stunde bei Raumtemperatur gerührt und danach mit 50 ml Wasser verdünnt. Der gebildete Festkörper wird abgetrennt und getrocknet, wobei man 1,3 g des Produkts als weissliche

Nadeln erhält, Schmelzpunkt 250 - 254°. Umkristallisation aus Aethanol liefert weisse Nadeln, Schmelzpunkt 252 - 254°.

## Beispiel 8

Herstellung von 1-[2-(4-Chlorphenyl)-4-chinolinyl]-N-methyl-4-piperidincarboxamid

(a) N-Methylisonipecotamid

Ein Gemisch von 20 g (0.147 Mol) N-Methylisonicotinamid, 100 ml Aethanol und 1.2 g Platinoxyd wird in einer Parr--Apparatur bei 60° und 0.425 MPa 8 Stunden hydriert. Der Katalysator wird abfiltriert, und das Filtrat unter vermindertem Druck eingedampft. Der Rückstand wird aus Aether kristallisiert, wobei man 20.5 g des rohen Produkts erhält, Schmelzpunkt 115 - 121°.

(b) 1-[2-(4-Chlorphenyl)-4-chinolinyl]-N-methyl-4-piperidincarboxamid

Ein Gemisch von 5.1 g (21 mMol) 4-Chlor-2-(4-chlorphenyl)chinolin (hergestellt in Beispiel 3 (a)), 6.2 g (42 mMol) N-Methylisonipecotamid und 10 g Phenol wird unter Rühren in einem Oelbad während 3 1/2 Stunden auf 160° erhitzt. Das Gemisch wird danach abgekühlt und mit 100 ml Wasser verdünnt. Beim Anreiben, Erwärmen und Zugeben einer geringen Menge Aethanol tritt Kristallisation ein. Der Festkörper wird abgetrennt und mit Aether gewaschen, wobei man 7.0 g Rohprodukt erhält. Umkristallisation aus 150 ml Butanol liefert 4.6 g des Produkts, Schmelzpunkt 151 - 155° (Zers.).

## Beispiel 9

Herstellung von 1-[2-(4-Chlorphenyl)-4-chinolinyl]-4-piperidincarboxamid

In analoger Weise wie in Beispiel 8 beschrieben, erhält man durch Umsetzen von 4-Chlor-2-(4-chlorphenyl)chinolin mit Isonipecotamid in Phenol bei 170° die obengenannte Verbindung, Schmelzpunkt 245 - 247° (Zers.) aus Aethanol.

## Beispiel 10

Herstellung von 1-[2-(4-Fluorphenyl)-4-chinolinyl]-4-piperidincarboxamid

(a) 2-(4-Fluorphenyl)-4-chinolincarbonsäure

Zu einer Suspension von 36.78 g (0.25 Mol) Isatin in 500 ml Aethanol werden 75 ml einer 10N Natriumhydroxydlösung gegeben. Nach wenigen Minuten Rühren fällt ein Festkörper aus. Zu diesem Gemisch werden 35 ml (0.286 Mol) p-Fluoracetophenon gegeben, und das Reaktionsgemisch unter Rühren während 1 1/2 Stunden zum Rückfluss erhitzt. Danach lässt man leicht abkühlen und säuert durch Zugabe von 250 ml 3N Salzsäure an. Der ausgefallene Festkörper wird abgetrennt, mit Wasser und mit Aether gewaschen und getrocknet, wobei man 50.6 g Rohprodukt erhält, Schmelzpunkt 205 - 215°. Umkristallisation aus Aethanol liefert 38.8 g der obengenannten Säure, Schmelzpunkt 210 - 213° (Zers.).

(b) 2-(4-Fluorphenyl)-4-chinolincarbonylchlorid

Ein Gemisch von 16.5 g 2-(4-Fluorphenyl)-4-chinolincarbonsäure, 100 ml Thionylchlorid und 50 ml Toluol wird unter Rühren während 4 Stunden zum Rückfluss erhitzt. Das Reaktionsgemisch wird danach unter vermindertem Druck eingedampft, der Rückstand mit neuem Toluol versetzt, und das Gemisch wiederum unter vermindertem Druck eingedampft. Der Rückstand wird mit Aether gewaschen, wobei man 16 g der obengenannten Verbindung als gelben Festkörper erhält.

(c) 4-Chlor-2-(4-fluorphenyl)chinolin

Ein Gemisch von 3g 2-(4-Fluorphenyl)-4-chinolincarbonylchlorid und 0.1 g tris-(Triphenylphosphin)rhodium-(1)-chlorid wird in einem Oelbad 40 Minuten auf 220° bis 235° erhitzt. Das Reaktionsgemisch wird danach abgekühlt und mit Methylenchlorid extrahiert. Die Extrakte werden durch 100 ml Silicagel filtriert, und das Silicagel mit weiterem Methylenchlorid gewaschen. Die Filtrate werden vereinigt und unter vermindertem Druck eingeengt, wobei man 0.9 g Rohprodukt erhält, Schmelzpunkt 90 - 93°. Umkristallisation aus Methanol liefert eine Analysenprobe vom Schmelzpunkt 91 - 93°.

(d) 1-[2-(4-Fluorphenyl)-4-chinolinyl]--4-piperidincarboxamid

Ein Gemisch von 1.44 g 4-Chlor-2-(4-fluorphenyl)-chinolin, 1.3 g (10mMol) Isonipecotamid und 3 g Phenol wird unter Rühren während 4 1/2 Stunden in einem Oelbad auf 165° bis 170° erhitzt. Das Gemisch wird danach abgekühlt, mit Wasser verdünnt und mit einer 3N Natriumhydroxydlösung alkalisch gestellt. Durch Zugabe von Aether und Anreiben tritt Kristallisation ein. Der Festkörper wird abgetrennt und mit Aether gewaschen, wobei man 1.6 g Rohprodukt erhält. Kristallisation aus Aethanol/Wasser liefert weisse Nadeln (0.42 g) vom Schmelzpunkt 218 - 220° (Zers.).

## Beispiel 11

Herstellung von 1-[6-Fluor-2-phenyl-4-chinolinyl]-4-piperidincarboxamid

(a) 6-Fluor-2-phenyl-4-chinolinol

Ein Gemisch von 100 g p-Fluoranilin, 160 ml Aethylbenzoylacetat, 200 ml Toluol und 4 g p-Toluolsulfonsäure

wird gerührt und mit einer Dean-Stark Wasserfalle zum Rückfluss erhitzt bis 15 ml Wasser abgeschieden sind. Das Reaktionsgemisch wird danach unter vermindertem Druck zu 275 g eines schwarzen Oels eingeengt. Das Oel wird in 500 ml Diphenyläther gegossen und auf 240° erhitzt. Nach 40 Minuten Erhitzen wird die Heizung entfernt, und das Reaktionsgemisch auf Raumtemperatur abgekühlt. Danach wird es mit Hexan verdünnt, und der gebildete Festkörper abgetrennt. Dieser Festkörper wird mit Hexan und Methylenchlorid gewaschen, wobei man 104.3 g 4-Fluor-2-phenyl-4-chinolinol erhält, Schmelzpunkt 298 - 302°.

(b) 4-Chlor-6-fluor-1-phenylchinolin

Ein Gemisch von 30.4 g (0.127 Mol)-6-Fluor-2-phenyl-4--chinolinol und 65 ml Phosphoroxychlorid wird unter Rühren während 2 Stunden zum Rückfluss erhitzt. Das Gemisch wird danach unter vermindertem Druck eingeengt, mit Methylenchlorid verdünnt und auf Eis gegossen. Die wässrige Phase wird mit 3N Natriumhydroxydlösung neutralisiert. Die organische Phase wird abgetrennt, und die wässrige Phase mit Methylenchlorid extrahiert. Die organischen Phasen werden vereinigt, mit Wasser und mit gesättigter Kochsalzlösung gewaschen, über Natriumsulfat getrocknet und unter vermindertem Druck eingedampft, wobei man 34.8 g Rückstand erhält. Dieser wird in Methylenchlorid gelöst, und die Lösung durch Aluminiumoxyd filtriert. Einengen des Filtrates liefert 23.7 g 4-Chlor-6-fluor-2-phenylchinolin, Schmelzpunkt 96 - 99°.

(c) 1-(2-Phenyl-6-fluor-4-chinolinyl) -4-piperidincarboxamid

Umsetzen von 4-Chlor-6-fluor-2-phenylchinolin und Isonipecotamid in analoger Weise wie in Beispiel 6 (c) beschrieben, liefert die obengenannte Verbindung in Form weisslicher Prismen, Schmelzpunkt 230 - 233°, nach Umkristallisation aus Aethanol.

## Beispiel 12

Herstellung von N-Aethyl-1-[6-fluor-2-phenyl-4-chinolinyl] -4-piperidincarboxamid

(a) 1-[6-Fluor-2-phenyl-4-chinolinyl] -4-piperidincarbonsäureäthylester

Ein Gemisch von 9.0 g 4-Chlor-6-fluor-2-phenylchinolin, das nach dem in Beispiel 11 (b) beschriebenen Verfahren hergestellt wurde, und 18 ml Aethylisonipecotat wird unter Rühren in einem Oelbad während 2 Stunden auf 160° bis 165° erhitzt. Das Gemisch wird danach abgekühlt und mit Wasser verdünnt. Bei der Zugabe von Aethanol fällt ein Festkörper aus, der abgetrennt wird und 7.2 g des obengenannten Produkts in Form weisser Prismen liefert, Schmelzpunkt 120 - 124°.

(b) 1-[6-Fluor-2-phenyl-4-chinolinyl]-4-piperidincarbonsäure

Aus 6.0 g 1-[6-Fluor-2-phenyl-4-chinolinyl] -4-piperidincarbonsäureäthylester und 10 ml 3N Natriumhydroxydlösung werden in analoger Weise wie in Beispiel 3 (c) beschrieben, 5.6 g des obengenannten Produkts als gelbe Nadeln erhalten, Schmelzpunkt 255 - 260° (Zers.).

(c) 1-[6-Fluor-2-phenyl-4-chinolinyl -4-piperidincarbonylchlorid

Ein Gemisch von 4 g 1-[6-Fluor-2-phenyl -4-chinolinyl]-4-piperidincarbonsäure, 12 ml Thionylchlorid und 100 ml Methylenchlorid wird unter Rühren während einer Stunde zum Rückfluss erhitzt. Das Reaktionsgemisch wird abgekühlt und unter vermindertem Druck eingeengt, wobei man 4.2 g des Produkts als gelben Festkörper erhält, Schmelzpunkt 243 - 245°.

(d) N-Aethyl-1-[6-fluor-2-phenyl-4-chinolinyl] -4-piperidincarboxamid

Unter Verwendung von 1-[6-Fluor-2-phenyl-4-chinolinyl] -4-piperidincarbonylchdlorid und Aethylamin als Ausgangsstoffe wird in analoger Weise wie in Beispiel 3 (e) beschrieben, die obengenannte Verbindung nach Umkristallisation aus Methanol als cremefarbene Nadeln erhalten, Schmelzpunkt 225 - 227° (Zers.).

## Beispiel 13

Herstellung von 1-[6-Fluor-2-phenyl-4-chinolinyl] -N-propyl-4-piperidincarboxamid

Unter Verwendung von 1-[6-Fluor-2-phenyl-4-chinolinyl] -4-piperidincarbonylchlorid und Propylamin als Ausgangsstoffe wird in analoger Weise wie in Beispiel 3 (e) beschrieben, die obengenannte Verbindung nach Umkristallisation aus Methanol als weissliche Nadeln erhalten, Schmelzpunkt 196 - 198° (Zers.).

## Beispiel 14

Herstellung von 1-[6-Fluor-2-(4-fluorphenyl)-4--chinolinyl]-4-piperidincarboxamid

(a) 6-Fluor-4-hydroxychinolin

Ein Gemisch von 216 ml Diäthyläthoxymethylenmalonat, 95 ml 4-Fluoranilin und 800 ml Diphenyläther wird gerührt und langsam auf 185° erhitzt, wobei das gebildete Aethanol abdestilliert wird. Das Reaktionsgemisch wird danach während 1 1/2 Stunden auf 245° erhitzt. Gesamthaft werden 105 ml Aethanol abgetrennt. Man lässt das Reaktionsgemisch danach auf 60° abkühlen, während man 250 ml Hexan zugibt. Nach Stehenlassen über Nacht wird das Produkt abgetrennt, mit

Hexan gewaschen und getrocknet, wobei man 223 g eines Festkörpers erhält, Schmelzpunkt 305 - 309°.

Ein Gemisch des obigen Festkörpers mit 600 ml 3N Natriumhydroxydlösung und 250 ml Wasser wird unter Rühren während 2 Stunden zum Rückfluss erhitzt. Zur heissen, braunen, trüben Mischung werden zur Ausfällung des Produkts 6-Fluor-4-hydroxychinolin-3-carbonsäure langsam 600 ml 3N Salzsäure gegeben. Dieses Produkt wird vom abgekühlten Reaktionsgemisch filtriert.

Dieser feuchte Festkörper und 600 ml Diphenyläther werden langsam auf 250° erhitzt, während man Wasser abdestillieren lässt. Das Reaktionsgemisch wird 20 Minuten bei dieser Temperatur gehalten, und danach lässt man auf 100° abkühlen. Dann werden 500 ml Hexan zugegeben, und das Gemisch während einer Stunde gerührt. Das Produkt 6-Fluor-4--hydroxychinolin wird abgetrennt und mit Hexan gewaschen, wo- bei man 144 g erhält, Schmelzpunkt 209 - 212°.

(b) 4-Chlor-6-fluorchinolin

Ein Gemisch von 52 g (0.319 Mol) 6-Fluor-4-hydroxychinolin und 100 ml Phosphoroxychlorid wird unter Rühren während 3 Stunden zum Rückfluss erhitzt. Das Reaktionsgemisch wird danach unter vermindertem Druck eingedampft. Ein Aufschlämmung des Rückstands in Methylenchlorid wird auf Eiswasser gegossen, und das Gemisch mit 3N Natriumhydroxydlösung neutralisiert. Die organische Phase wird abgetrennt, und die wässrige Phase mit 250 ml Methylenchlorid zweimal extrahiert. Die organischen Phasen werden vereinigt, mit Wasser gewaschen, über Natriumsulfat getrocknet und unter vermindertem Druck eingedampft. Der Rückstand wird aus Hexan kristallisiert, wobei man 27.95 g des Produkts erhält, Schmelzpunkt 71 - 74°.

(c) 4-Chlor-6-fluor-2-(4-fluorphenyl)chinolin

Zu einer auf -10° gekühlten Lösung von 4.37 g (26 mMol) 1-Brom-4-fluorbenzol und 35 ml Aether werden 15 ml einer 1.6M Lösung von Butyllithium in Hexan portionenweise gegeben, wobei man die Temperatur unterhalb 0° hält. Das Reaktionsgemisch wird während 15 Minuten unterhalb von 0° gerührt. Eine Lösung von 3.63 g (20 mMol) 4-Chlor-6-fluorchinolin in 20 ml Tetrahydrofuran wird zugegeben, wobei man die Temperatur unterhalb 0° hält. Die dunkle Lösung wird 20 Minuten bei Raumtemperatur gerührt. Zur Reaktionsmischung werden dann hintereinander 5ml Wasser, 5g Jod und 10 ml 3N Natriumhydroxydlösung gegeben. Nach 10minütigem Rühren wird der Festkörper abgetrennt, wobei man 2.8 g des Produkts erhält, Schmelzpunkt 130 - 133°.

(d) 1-[6-Fluor-2-(4-fluorphenyl)-4-chinolinyl] -4-piperidincarboxamid

Die obengenannte Verbindung wurde in analoger Weise wie in Beispiel 6 (c) beschrieben unter Verwendung von 4-Chlor-6-fluor-2-(4-fluorphenyl)chinolin und Isonipecotamid als Ausgangsstoffe hergestellt. Nach Umkristallisation aus Aethanol wird das Produkt in Form weisslicher Nadeln vom Schmelzpunkt 244 - 246° (Zers.) erhalten.

## Beispiel 15

Herstellung von 1-[6-Fluor-2-(4-fluorphenyl)-4--chinolinyl)-N-methyl -4-piperidincarboxamid

Die obengenannte Verbindung wurde in analoger Weise wie in Beispiel 6 (c) beschrieben unter Verwendung von 4-Chlor-6-fluor-2-(4-fluorphenyl)chinolin (aus Beispiel 14 (c)) und N-Methylisonipecotamid als Ausgangsstoffe hergestellt. Nach Umkristallisation aus Acetonitril wird das Produkt in Form weisser Prismen vom Schmelzpunkt 256 - 259°

erhalten.

## Beispiel 16

Herstellung von N-Aethyl-1-[6-fluor-2-(4-fluorphenyl) -4-chinolinyl]-4-piperidincarboxamid

In analoger Weise wie in Beispiel 3 beschrieben, wurde die obengenannte Verbindung unter Verwendung von 1-[6-Fluor-2-phenyl-4-chinolinyl] -4-piperidincarbonylchlorid (aus Beispiel 12 (c)) und Aethylamin als Ausgangsstoffe hergestellt. Nach Umkristallisation aus Aethanol wird das Produkt in Form weisser Prismen vom Schmelzpunkt 236 - 239° erhalten.

## Beispiel 17

Herstellung von 1-[2-(4-Methylphenyl)-4-chinolinyl] -4-piperidincarboxamid

(a) 4-Chlor-2-(4-methylphenyl)chinolin

Zu einer auf 5° abgekühlten Lösung von 4.3 g (25 mMol) p-Bromtoluol in 75 ml Aether werden tropfenweise 15 ml einer 1.6M Lösung von Butyllithium in Hexan gegeben, wobei man die Temperatur unterhalb 5° hält. Dieses Gemisch wird dann während 10 Minuten bei 5° und während 10 Minuten bei 30° gerührt und danach auf -20° abgekühlt. Eine Lösung von 3.4 g (20 mMol) 4-Chlorchinolin in 15 ml Aether wird zugegeben, wobei man die Temperatur bei -20° hält. Danach lässt man während 15 Minuten auf Raumtemperatur erwärmen. Dann werden nacheinander 10 ml Wasser, 5 g Jod und 60 ml 3N Natriumhydroxydlösung zum Reaktionsgemisch gegeben. Nach 20minütigem Rühren wird die organische Phase abgetrennt. Die wässrige Phase wird mit dem gleichen Volumen Aether in 3 Portionen extrahiert. Die organischen Phasen werden vereinigt, mit Wasser gewaschen, über Natriumsulfat getrocknet und unter

vermindertem Druck zu einem Oel eingedampft. Dieses Oel wird in Methylenchlorid gelöst und durch Aluminiumoxyd filtriert. Das Filtrat wird unter vermindertem Druck eingedampft, wobei man 2.6 g des Produkts als weissliche Nadeln erhält, Schmelzpunkt 75 - 77°.

(b) 1-[2-(4-Methylphenyl)-4-chinolinyl] -4-piperidincarboxamid

In analoger Weise wie in Beispiel 3 (b) beschrieben, wurde unter Verwendung von 4-Chlor-2-(4-methylphenyl)-chinolin und Isonipecotamid als Ausgangsstoffe das obengenannte Produkt hergestellt, welches nach Umkristallisation aus Acetonitril einen Schmelzpunkt von 230 - 232° aufweist.

Beispiel 18

Herstellung von 1-[2-(4-Bromphenyl)-4-chinolinyl] -4-piperidincarboxamid

(a) 2-(4-Bromphenyl)-4-chinolincarbonsäure

Umsetzen von 29.4 g Isatin, 47.8 g p-Bromacetophenon und 60 ml 10N Natriumhydroxydlösung in 400 ml Aethanol nach dem Verfahren gemäss Beispiel 10 (a) ohne Umkristallisation liefert 62.5 g 2-(4-Bromphenyl)-4-chinolincarbonsäure als weissliche Prismen, Schmelzpunkt 238 - 240° (Zers.).

(b) 2-(4-Bromphenyl)-4-chinolincarbonylchlorid

Aus 20 g 2-(4-Bromphenyl)-4-chinolincarbonsäure werden durch Umsetzen mit Thionylchlorid gemäss dem Verfahren von Beispiel 10 (b) 16 g 2-(4-Bromphenyl)-4-chinolincarbonylchlorid als gelbe Prismen erhalten, Schmelzpunkt 139 - 142° (Zers.).

(c) 2-(4-Bromphenyl)-4-chlorchinolin

Umsetzen von 3 g 2-(4-Bromphenyl)-4-chinolincarbonylchlorid mit tris-(Triphenylphosphin)rhodium-(1)-chlorid gemäss dem Verfahren von Beispiel 10 (c) liefert nach Filtrieren einer Methylenchloridlösung durch Aluminiumoxyd 2.2 g 2-(4-Bromphenyl)-4-chlorchinolin, Schmelzpunkt 115 - 177° (Zers.).

(d) 1-[2-(4-Bromphenyl)-4-chinolinyl]-4-piperidincarboxamid

Die obengenannte Verbindung wurde in analoger Weise wie in Beispiel 10 (d) beschrieben unter Verwendung von 2-(4-Bromphenyl)-4-chlorchinolin und Isonipecotamid als Ausgangsstoffe hergestellt. Umkristallisation aus Aethanol liefert das Produkt in Form weisser Nadeln vom Schmelzpunkt 247 - 249° (Zers.).

## Beispiel 19

Herstellung von 1-[6-Fluor-2-(4-methylphenyl) -4-chinolinyl]-4-piperidincarboxamid

(a) 4-Chlor-6-fluor-2-(4-methylphenyl)chinolin

Zu einer auf 5° abgekühlten Lösung von 4.3 g (25mMol) p-Bromtoluol in 75 ml Aether werden tropfenweise 10 ml einer 1.6M Lösung von Butyllithium in Hexan gegeben, während man die Temperatur unterhalb 5° hält. Das Gemisch wird 10 Minuten bei 5° und 10 Minuten bei 30° gerührt und danach auf -20° gekühlt. Eine Lösung von 3.63 g (20 mMol) 4-Chlor-6--fluorchinolin (aus Beispiel 14 (b)) in 20 ml Aether wird zugegeben, während man die Temperatur bei -20° hält. Das Reaktionsgemisch wird danach bei Raumtemperatur 15 Minuten gerührt. Zur Reaktionsmischung werden danach nacheinander 10 ml Wasser, 5 g Jod und 60 ml 3N Natriumhydroxydlösung gegeben. Nach 20minütigem Rühren wird die organische Phase

abgetrennt. Die wässrige Phase wird mit einem gleichen Volumen Aether extrahiert. Die organischen Phasen werden vereinigt, mit Wasser gewaschen, über Natriumsulfat getrocknet und unter vermindertem Druck eingedampft, wobei man 5.8 g des Rohprodukts als blassoranges Oel erhält.

(b) 1-[6-Fluor-2-(4-methylphenyl)-4-chinolinyl] -4-piperidincarboxamid

Die obengenannte Verbindung wurde in analoger Weise wie in Beispiel 6 (c) beschrieben, unter Verwendung von 4-Chlor-6-fluor-2-(4-methylphenyl)chinolin und Isonipectoamid als Ausgangsstoffe hergestellt. Nach Umkristallisation aus Pyridin wird das Produkt in Form weisser Nadeln vom Schmelzpunkt 246 - 249° (Zers.) erhalten.

## Beispiel 20

Herstellung von 1-[6-Chlor-2-(4-methylphenyl)-4--chinolinyl] -4-piperidincarboxamid

(a) 6-Chlor-2-(4-methylphenyl)-4-chinolincarbonsäure

Zu einer Suspension von 18.15 g 5-Chlorisatin in 200 ml Aethanol werden 30 ml 10N Natriumhydroxydlösung gegeben. Zu diesem Gemisch werden danach 16 ml (0.12 Mol) p-Methylacetophenon gegeben, und das Gemisch während 3.2 Stunden unter Rühren zum Rückfluss erhitzt. Danach wird unter vermindertem Druck eingeengt, und der Rückstand mit 250 ml Wasser verdünnt. Die obengenannte Säure wird durch die Zugabe von Essigsäure als bräunlicher Festkörper ausgefällt. Umkristallisation aus Acetonitril liefert 31.6 g der obengenannten Säure als bräunlichen Festkörper.

(b) 6-Chlor-2-(4-methylphenyl)-4-chinolincarbonylchlorid

Ein Gemisch von 28.4 g 6-Chlor-2-(4-methylphenyl) -4-chinolincarbonsäure, 100 ml Thionylchlorid und 200 ml Toluol wird unter Rühren während 1 1/2 Stunden zum Rückfluss erhitzt. Das Reaktionsgemisch wird danach unter vermindertem Druck eingeengt, der Rückstand mit neuem Toluol versetzt und wiederum unter vermindertem Druck eingeengt. Der Rückstand wird aus 300 ml Heptan umkristallisiert, wobei man 17.7 g der obengenannten Verbindung als gelblichen Festkörper erhält.

(c) 4.6-Dichlor-2-(4-methylphenyl)chinolin

Ein Gemisch von 3 g 6-Chlor-2-(4-methylphenyl)-4--chinolincarbonylchlorid und 0.2 g tris-(Triphenylphosphin)-rhodium-(1)-chlorid wird während 40 Minuten in einem Oelbad auf 180° bis 200° erhitzt. Das Reaktionsgemisch wird danach abgekühlt und mit Aethylenchlorid extrahiert. Die Extrakte werden durch 75 ml Aluminiumoxyd filtriert, und das Aluminiumoxyd mit weiterem Methylenchlorid nachgewaschen. Die Filtrate werden vereinigt und unter vermindertem Druck eingeengt, wobei man 0.8 g des Rohprodukts erhält, Schmelzpunkt 123 - 125°.

(d) 1-[6-Chlor-2-(4-methylphenyl)-4-chinolinyl] -4-piperidincarboxamid

Ein Gemisch von 2.02 g (7 mMol) 4,6-Dichlor-2-(4-methylphenyl)chinolin, 1.6 g (12 mMol) Isonipecotamid, 5 g Phenol und geringe Mengen Natriumjodid und Kupferpulver werden unter Rühren 4 Stunden auf 160° erhitzt. Das Reaktionsgemisch wird dananch mit 50 ml Wasser verdünnt und durch Zugabe von konzentrierter Ammoniumhydroxydlösung alkalisch gestellt. Durch Zugabe von Aethylacetat und Aether kristallisieren 2.7 g eines rohen, feuchten Produkts vom Schmelzpunkt 260 - 265°. Umkristallisation aus Aethanol liefert die

obengenannte Verbindung in Form weisser Nadeln vom Schmelzpunkt 265 - 267° (Zers.).

Beispiel 21

Herstellung von 1-[2-(4-Chlorphenyl)-6-fluor -4-chinolinyl]-N-methyl-4-piperidincarboxamid

Die obengenannte Verbindung wurde in analoger Weise wie in Beispiel 6 (c) beschrieben, unter Verwendung von 4-Chlor-2-(4-chlorphenyl)-6-fluorchinolin (aus Beispiel 6 (b)) N-Methylisonipecotamid als Ausgangsstoffe hergestellt. Nach Umkristallisation aus Pyridin wird das Produkt in Form farbloser Nadeln vom Schmelzpunkt 279 - 282° (Zers.) erhalten.

Beispiel 22

Herstellung von 1-[6-Chlor-2-(4-fluorphenyl)-4-chinolinyl] -4-piperidincarboxamid

(a) 6-Chlor-4-hydroxychinolin-3-carbonsäureäthylester

Aus einem Gemisch von 216 g Diäthyläthoxymethylenmalonat, 127.6 g p-Chloranilin und 800 ml Diphenyläther, welches zunächst während 15 Minuten auf 150 bis 160° und danach während 20 Minuten auf 245° erhitzt wird, erhält man nach Abkühlen die obengenannte Verbindung als Niederschlag. Dieser Niederschlag wird abgetrennt und mit Wasser und Hexan gewaschen, wobei man 245 g eines rohen Festkörpers erhält, welcher direkt in die weiter unten beschriebene Hydrolysereaktion eingesetzt wird.

(b) 6-Chlor-4-hydroxychinolin

Aus 6-Chlor-4-hydroxychinolin-3-carbonsäureäthylester erhält man in analoger Weise wie in Beispiel 14 beschrieben,

nach Umkristallisation aus Aethanol die obengenannte Verbindung vom Schmelzpunkt 265 - 268°.

(c) 4,6-Dichlorchinolin

Durch Umsetzen von 93.6 g 6-Chlor-4-hydroxychinolin mit 200 ml Phosphoroxychlorid in analoger Weise wie in Beispiel 4 (b) beschrieben, erhält man 63.6 g der obengenannten Verbindung als weisse Nadeln, Schmelzpunkt 101 - 104°.

(d) 4,6-Dichlor-2-(4-fluorphenyl)chinolin

Zu einer auf -40° abgekühlten Lösung von 5.5 ml (50 mMol) 1-Brom-4-fluorbenzol in 70 ml Aether werden 21 ml einer 2.4M Lösung Butyllithium in Hexan gegeben, während man die Temperatur unterhalb -15° hält. Eine Lösung von 7.42 g (40 mMol) 4,6-Dichlorchinolin in 40 ml Tetrahydrofuran wird zugegeben, während man die Temperatur bei -20° hält. Danach lässt man während 15 Minuten auf 0° erwärmen. Zum Reaktionsgemisch werden dann nacheinander 10 ml Wasser, 10.16 g (40 mMol) Jod und 15 ml 3N Natriumhydroxydlösung gegeben. Nach 10minütigem Rühren wird die Mischung auf 5° abgekühlt, und der Festkörper abgetrennt. Der Festkörper wird in Methylenchlorid gelöst, und die Lösung durch Aluminiumoxyd filtriert. Das Filtrat wird unter vermindertem Druck eingeengt, und der erhaltene Rückstand aus Aethylacetat umkristallisiert, wobei man 5.7 g des Rohprodukts erhält, Schmelzpunkt 150 - 156°.

(e) 1-[6-Chlor-2-(4-fluorphenyl)-4-chinolinyl] -4-piperidincarboxamid

In analoger Weise wie in Beispiel 6 (c) beschrieben, erhält man aus 4,6-Dichlor-2-(4-fluorphenyl)chinolin und Isonipecotamid und Umkristallisation aus Aethanol die

obengenannte Verbindung in Form weisser Nadeln, Schmelzpunkt 250 - 252° (Zers.).

## Beispiel 23

Herstellung von 1-[6-Chlor-2-(4-fluorphenyl) -4-chinolinyl]-N-methyl-4-piperidincarboxamid

Die obengenannte Verbindung wurde in analoger Weise wie in Beispiel 6 (c) beschrieben, ausgehend von 4,6-Dichlor-2-(4-fluorphenyl)chinolin (aus Beispiel 22) und N-Methylisonipecotamid hergestellt. Nach Umkristallisation aus Pyridin/Wasser wird die Verbindung in Form weisslicher Nadeln erhalten, Schmelzpunkt 260 - 265° (Zers.).

## Beispiel 24

Herstellung von 1-[6-Chlor-2-(4-chlorphenyl) -4-chinolinyl]-4-piperidincarboxamid

(a) 4,6-Dichlor-2-(4-chlorphenyl)chinolin

Zu einer auf -10° gekühlten Lösung von 4.78 g (25 mMol) 1-Brom-4-chlorbenzol in 40 ml Aether werden 15 ml einer 2.4M Lösung von Butyllithium in Hexan gegeben, während man die Temperatur unterhalb 0° hält. Das Reaktionsgemisch wird 15 Minuten bei 0° gerührt. Während man die Temperatur bei 0° hält, wird eine Lösung von 3.96 g (20 mMol) 4,6-Dichlorchinolin, hergestellt wie in Beispiel 22 (c) beschrieben, in 20 ml Tetrahydrofuran zugegeben. Man rührt dann 25 Minuten bei 0° und gibt danach nacheinander 10 ml Wasser, 5 g Jod und 50 ml 3N Natriumhydroxydlösung zum Reaktionsgemisch. Nach 10minütigem Rühren wird das Gemisch auf 5° gekühlt, und der Festkörper abgetrennt. Waschen des Festkörpers mit Aether und Trocknen liefert 6.23 g der obengenannten Verbindung, Schmelzpunkt 204 - 208°.

(b) 1-[6-Chlor-2-(4-chlorphenyl)-4-chinolinyl] -4-piperidincarboxamid

Ein Gemisch von 3.1 g 4,6-Dichlor-2-(4-chlorphenyl)-chinolin, 2.56 g (20 mMol) Isonipecotamid und 10 g Phenol wird unter Rühren während 4 Stunden in einem Oelbad auf 160° bis 170° erhitzt. Danach wird es abgekühlt und mit Wasser verdünnt. Das Gemisch wird dann mit 3N Natriumhydroxydlösung alkalisch gestellt, wobei nach Zugabe von Aether und Anreiben Kristallisation eintritt. Der Festkörper wird abgetrennt und mit Aether gewaschen, wobei man 2.45 g des Rohprodukts erhält. Umkristallisation aus Aethanol liefert die obengenannte Verbindung in Form weisslicher Prismen, Schmelzpunkt 272 - 275°.

## Beispiel 25

Herstellung von 1-[6-Chlor-2-phenyl-4-chinolinyl] -4-piperidincarboxamid

(a) 6-Chlor-2-phenyl-4-chinolinol

Aus 100 g p-Chloranilin und 138.5 ml Aethylbenzoylacetat wurde in analoger Weise wie in Beispiel 6 (a) beschrieben 115.6 g 6-Chlor-2-phenyl-4-chinolinol erhalten, Schmelzpunkt >350°.

(b) 4,6-Dichlor-2-phenylchinolin

Aus 25.5 g 6-Chlor-2-phenyl-4-chinolinol und 50 ml Phosphoroxychlorid wurde in analoger Weise wie in Beispiel 6 (b) beschrieben 24.86 g 4,6-Dichlor-2-phenylchinolin erhalten, Schmelzpunkt 114 - 116°.

(c) 1-[6-Chlor-2-phenyl-4-chinolinyl]-4-piperidincarboxamid

Die obengenannte Verbindung wurde in analoger Weise wie in Beispiel 6 (c) beschrieben, unter Verwendung von 4,6-Dichlor-2-phenylchinolin und Isonipecotamid als Ausgangsstoffe hergestellt. Die Verbindung wird nach Umkristallisation aus Aethanol in Form weisser Nadeln erhalten, Schmelzpunkt 253 - 255° (Zers.).

## Beispiel 26

Herstellung von 1-[6-Methyl-2-phenyl-4-chinolinyl] -4-piperidincarboxamid

(a) 4-Chlor-6-methyl-2-phenylchinolin

Diese Verbindung wurde in analoger Weise wie in Beispiel 6 beschrieben, ausgehend von p-Toluidin und Aethylbenzoylacetat, hergestellt und in Form eines leicht gefärbten Festkörpers erhalten, Schmelzpunkt 90 - 93°.

(b) 1-[6-Methyl-2-phenyl-4-chinolinyl]-4-piperidincarboxamid

Die obengenannte Verbindung wurde in analoger Weise wie in Beispiel 6 (c) beschrieben, unter Verwendung von 4-Chlor-6-methyl-2-phenylchinolin und Isonipecotamid als Ausgangsstoffe hergestellt. Nach Umkristallisation aus Pyridin wird die Verbindung in Form eines weissen Festkörpers erhalten, Schmelzpunkt 245 - 247° (Zers.).

## Beispiel 27

Herstellung von 1-[2-(4-Fluormethyl)-6-methyl-4- -chinolinyl -4-piperidincarboxamid

(a) 4-Chlor-6-methylchinolin

Diese Verbindung wurde in analoger Weise zur in Beispiel 14 beschriebenen Herstellung von 4-Chlor-6-fluorchinolin erhalten. Ausgehend aus p-Toluidin und Diäthyläthoxymethylenmalonat wurde die Verbindung als Festkörper vom Schmelzpunkt 47 - 51° erhalten.

(b) 4-Chlor-2-(4-fluorphenyl)-6-methylchinolin

Diese Verbindung wird in analoger Weise zur in Beispiel 22 beschriebenen Synthese von 4,6-Dichlor-2-(4--fluorphenyl)chinolin hergestellt. Ausgehend von 3.5 ml 1-Brom-4-fluorbenzol und 4.3 g 4-Chlor-6-methylchinolin werden nach Filtrieren der Lösung des Produktes in Methylenchlorid durch Silicagel 2 g der obengenannten Verbindung als weisser Festkörper erhalten, Schmelzpunkt 93 - 95°.

(c) 1-[2-(4-Fluorphenyl)-6-methyl-4-chinolinyl] -4-piperidincarboxamid

Ein Gemisch von 2.0 g 4-Chlor-2-(4-fluorphenyl)-6--methylchinolin, 1.88 g (10mMol) Isonipecotamid und 4 g Phenol wird während 2 Stunden unter Rühren in einem Oelbad auf 165° bis 170° erhitzt. Danach wird es abgekühlt und mit Wasser verdünnt. Durch Zugabe von Aethanol und Anreiben tritt Kristallisation ein. Der Festkörper wird abgetrennt und mit Aether gewaschen, wobei man 1,9 g des Rohprodukts erhält, Schmelzpunkt 264 - 271° (Zers.). Umkristallisation aus Pyridin liefert die obengenannte Verbindung in Form weisslicher Prismen, Schmelzpunkt 275 - 277° (Zers.).

## Beispiel 28

Herstellung von 1-[6-Methyl-2-(4-methylphenyl) -4-chinolinyl]-4-piperidincarboxamid

(a) 4-Chlor-6-methyl-2-(4-methylphenyl)chinolin

Zu einer auf 5° gekühlten Lösung von 4.3 g (25 mMol) p-Bromtoluol in 50 ml Aether werden tropfenweise 15 ml einer 1.6M Lösung von Butyllithium in Heptan gegeben, während man die Temperatur unterhalb 5° hält. Das Reaktionsgemisch wird 10 Minuten bei 5° und 10 Minuten bei 30° gerührt und danach auf -20° abgekühlt. Eine Lösung von 4.0 g (22 mMol) 4-Chlor-6-methylchinolin, hergestellt wie in Beispiel 27 beschrieben, in 20 ml Aether wird zugegeben, während die Temperatur bei -20° gehalten wird. Danach lässt man 15 Minuten bei Raumtemperatur rühren. Zur Reaktionsmischung werden dann nacheinander 10 ml Wasser, 5 g Jod und 60 ml 3N Natriumhydroxydlösung gegeben. Nach 20 Minuten Rühren wird die organische Phase abgetrennt. Die wässrige Phase wird mit dem gleichen Volumen Aether extrahiert. Die organischen Phasen werden vereinigt, mit Wasser gewaschen, über Natriumsulfat getrocknet und unter vermindertem Druck eingeengt, wobei man das obengenannte 4-Chlorchinolin als gelbes Oel erhält. Eine Lösung dieses Oels in Methylenchlorid wird durch Aluminiumoxyd filtriert. Das Filtrat wird unter vermindertem Druck eingedampft, wobei man 3.22 g der obengenannten Verbindung erhält, Schmelzpunkt 40 - 50°.

(b) 1-[6-Methyl-2-(4-methylphenyl)-4-chinolinyl] -4-piperidincarboxamid

Ein Gemisch von 3.2 g 4-Chlor-6-methyl-2-(4-methylphenyl)chinolin, 3.06 g (24 mMol) Isonipecotamid und 6 g Phenol wird während 4 Stunden in einem Oelbad auf 160° erhitzt. Danach kühlt man ab und verdünnt mit Wasser. Durch Zugabe von Aethanol und Anreiben tritt Kristallisation ein. Der Festkörper wird abgetrennt und mit Aether gewaschen, wobei man 3.18 g Rohprodukt erhält. Umkristallisation aus n-Butanol liefert weissliche Prismen, Schmelzpunkt 242 - 244° (Zers.).

Beispiel 29

Herstellung von 1-[2-(4-Chlorphenyl)-6-methyl-4--chinolinyl] -4-piperidincarboxamid

(a) 4-Chlor-2-(4-chlorphenyl)-6-methylchinolin

Die obengenannte Verbindung wurde in analoger Weise wie in Beispiel 11 beschrieben, unter Verwendung von p-Toluidin und Aethyl p-chlorbenzoylacetat als Ausgangsstoffe hergestellt, Schmelzpunkt 136 - 138°.

(b) 1-[2-(4-Chlorphenyl)-6-methyl-4-chinolinyl] -4-piperidincarboxamid

Ein Gemisch von 10 g (35 mMol) 4-Chlor-2-(4-chlorphenyl)-6-methylchinolin, 8.9 g (70 mMol) Isonipecotamid und 20 g Phenol werden während 3 1/2 Stunden unter Rühren in einem Oelbad auf 165° bis 170° erhitzt. Danach wird abgekühlt und mit Wasser verdünnt. Durch Zugabe von Aethanol und Anreiben tritt Kristallisation ein. Der Festkörper wird abgetrennt und mit Aether gewaschen, wobei man 11.9 g Rohprodukt erhält. Umkristallisation aus Pyridin liefert die obengenannte Verbindung in Form weisslicher Prismen, Schmelzpunkt 261 - 265° (Zers.).

Beispiel 30

Herstellung von 1-[2-(4-Chlorphenyl)-4-chinolinyl] -N,N-dimethyl-4-piperidincarboxamid

In analoger Weise wie in Beispiel 3 (e) beschrieben, jedoch unter Verwendung von Dimethylamin anstelle von Aethylamin, wurde die obengenannte Verbindung hergestellt, Schmelzpunkt 157 - 159° (Zers.) aus Aethanol.

Beispiel 31

Aus den folgenden erfindungsgemässen Verbindungen der Formel I können verschiedene Formulierungsformen hergestellt werden:

1-[2-(4-Chlorphenyl)-4-chinolinyl] -N-äthyl-4-piperidincarboxamid,

1-[2-(4-Chlorphenyl)-6-fluor-4-chinolinyl] -4-piperidincarboxamid,

1-[2-(4-Chlorphenyl)-4-chinolinyl]-N-methyl -4-piperidincarboxamid und

1-[2-(4-Chlorphenyl)-4-chinolinyl] -4-piperidincarboxamid.

(a) Tablettenformulierung (direkte Verpressung)

| | mg/Tablette | | | |
|---|---|---|---|---|
| 1) Verbindung der Formel I | 1.0 | 5.0 | 10.0 | 50.0 |
| 2) Wasserfreier Milchzucker | 127.0 | 142.5 | 182.0 | 206.0 |
| 3) Mikrokristalline Cellulose | 40.0 | 50.0 | 60.0 | 80.0 |
| 4) Modifizierte Stärke | 10.0 | 12.5 | 15.0 | 20.0 |
| 5) Maisstärke | 20.0 | 25.0 | 30.0 | 40.0 |
| 6) Magnesiumstearat | 2.0 | 2.5 | 3.0 | 4.0 |
| Total | 200 mg | 250 mg | 300 mg | 400 mg |

Verfahren:

Die Bestandteile 1 bis 5 werden in einem geeigneten Mischer während 1 bis 15 Minuten vermischt. Der Bestandteil 6 wird zugegeben und gut vermischt. Danach wird auf einer geeigneten Presse zu Tabletten verpresst.

(b) Kapselformulierung

| | mg/Kapsel | | | |
|---|---|---|---|---|
| 1) Verbindung der Formel I | 1.0 | 5.0 | 10.0 | 50.0 |
| 2) Milchzucker | 149.0 | 182.5 | 215.0 | 250.0 |
| 3) Maisstärke | 40.0 | 50.0 | 60.0 | 80.0 |
| 4) Magnesiumstearat | 2.0 | 2.5 | 3.0 | 4.0 |
| 5) Talk | 8.0 | 10.0 | 12.0 | 16.0 |
| Total | 200 mg | 250 mg | 300 mg | 400 mg |

Verfahren:

Die Bestandteile 1 bis 3 werden in einem geeigneten Mixer vermischt und durch eine geeignete Mühle gemahlen. Die Bestandteile 4 und 5 werden zugegeben, worauf man nochmals vermischt. Danach wird auf einer geeigneten Maschine in Kapseln abgefüllt.

(c) Tablettenformulierung (feuchte Granulation)

| | mg/Tablette | | | |
|---|---|---|---|---|
| 1) Verbindung der Formel I | 1.0 | 5.0 | 10.0 | 50.0 |
| 2) Milchzucker | 195.0 | 230.0 | 264.0 | 263.0 |
| 3) Vorgelatinisierte Stärke | 12.5 | 15.0 | 17.5 | 20.0 |
| 4) Maisstärke | 25.0 | 30.0 | 35.0 | 40.0 |
| 5) Modifizierte Stärke | 12.5 | 15.0 | 17.5 | 20.0 |
| 6) Magnesiumstearat | 4.0 | 5.0 | 6.0 | 7.0 |
| Total | 200 mg | 300mg | 350 mg | 400 mg |

Verfahren:

Die Bestandteile 1 bis 5 werden in einem geeigneten Mixer vermischt, danach wird das Ganze mit Wasser zu einem Granulat verarbeitet. Nach dem Trocknen in einem geeigneten Ofen über Nacht wird das Ganze gemahlen und mit Bestandteil 6 vermischt. Danach wird auf einer geeigneten Presse zu Tabletten verpresst.

## Patentansprüche

1. 4-Amino-2-phenylchinoline der allgemeinen Formel

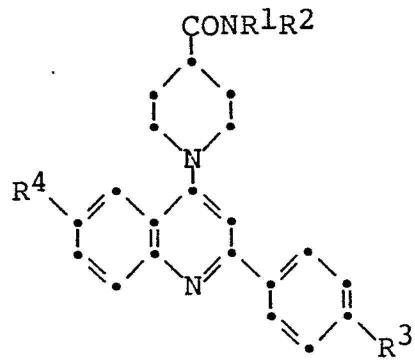

I

worin $R^1$ und $R^2$ unabhängig voneinander Wasserstoff oder nieder Alkyl und $R^3$ und $R^4$ unabhängig voneinander Wasserstoff, Halogen oder nieder Alkyl bedeuten, mit der Auflage, dass $R^3$ und $R^4$ nicht gleichzeitig Wasserstoff bedeuten.

2. Verbindungen gemäss Anspruch 1, worin $R^1$ und $R^2$ unabhängig voneinander Wasserstoff, Methyl oder Aethyl bedeuten.

3. Verbindungen gemäss Anspruch 1 oder 2, worin $R^3$ Chlor, Brom, Methyl oder Aethyl bedeutet.

4. 1-[2-(4-Chlorphenyl)-4-chinolinyl]-N-äthyl-4- piperidincarboxamid.

5. 1-[2-(4-Chlorphenyl)-6-fluor-4-chinolinyl]-4- piperidincarboxamid.

6. 1-[2-(4-Chlorphenyl)-4-chinolinyl]-N-methyl-4- piperidincarboxamid.

7. 1-[2-(4-Chlorphenyl)-4-chinolinyl]-4-piperidincarboxamid.

8. Verbindungen der allgemeinen Formel

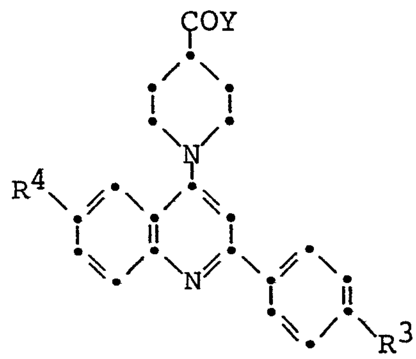

IV

worin Y Halogen, Hydroxy oder nieder Alkoxy und $R^3$ und $R^4$ unabhängig voneinander Wasserstoff, Halogen oder nieder Alkyl bedeuten, mit der Auflage, dass $R^3$ und $R^4$ nicht gleichzeitig Wasserstoff bedeuten.

9. 4-Amino-2-phenylchinoline gemäss einem der Ansprüche 1 bis 7 zur Anwendung als therapeutische Wirkstoffe.

10. 4-Amino-2-phenylchinoline gemäss einem der Ansprüche 1 bis 7 zur Anwendung bei der Bekämpfung bzw. Verhütung von Krampfanfällen und Angstzuständen.

11. Verfahren zur Herstellung einer Verbindung gemäss einem der Ansprüche 1 bis 7, dadurch gekennzeichnet, dass man

(a) ein 4-Halo-2-phenylchinolin der allgemeinen Formel

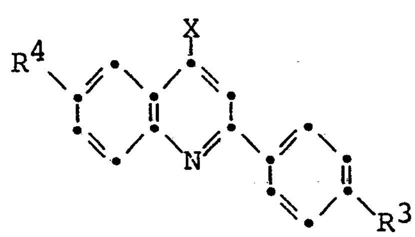

II

worin $R^3$ und $R^4$ die in Anspruch 1 angegebene Bedeutung besitzen und X Halogen bedeutet,

mit einem Isonipecotamid der allgemeinen Formel

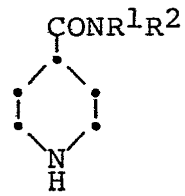

III

worin $R^1$ und $R^2$ die in Anspruch 1 angegebene Bedeutung besitzen,

umsetzt,

oder

(b) eine Verbindung der allgemeinen Formel

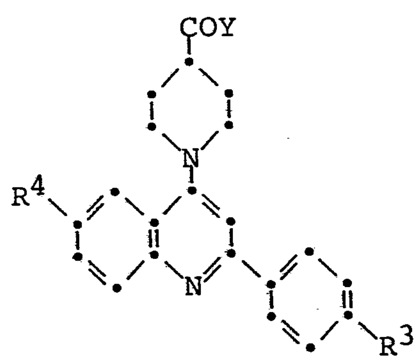

IV

worin $R^3$ und $R^4$ die in Anspruch 1 angegebene Bedeutung besitzen und Y Halogen, Hydroxy oder nieder Alkoxy bedeutet,

mit einem Amin der allgemeinen Formel

$$NHR^1R^2 \qquad V$$

worin $R^1$ und $R^2$ die in Anspruch 1 angegebene Bedeutung besitzen,

umsetzt.

12. Arzneimittel, enthaltend ein 4-Amino-2-phenylchinolin gemäss einem der Ansprüche 1 bis 7 und ein therapeutisch inertes Excipiens.

13. Mittel zur Bekämpfung bzw. Verhütung von Krampfanfällen und Angstzuständen, enthaltend ein 4-Amino-2-phenylchinolin gemäss einem der Ansprüche 1 bis 7 und ein therapeutisch inertes Excipiens.

14. Verwendung eines 4-Amino-2-phenylchinolins gemäss einem der Ansprüche 1 bis 7 bei der Bekämpfung bzw. Verhütung von Krankheiten.

15. Verwendung eines 4-Amino-2-phenylchinolins gemäss einem der Ansprüche 1 bis 7 bei der Bekämpfung bzw. Verhütung von Krampfanfällen und Angstzuständen.

16. Verwendung eines 4-Amino-2-phenylchinolins gemäss einem der Ansprüche 1 bis 7 zur Herstellung eines Mittels gegen Krampfanfälle und/oder Angstzustände.

***

Patentansprüche für Oesterreich

1. Verfahren zur Herstellung von 4-Amino-2-phenylchinolinen der allgemeinen Formel

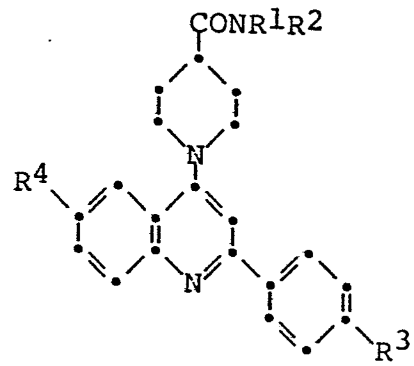

I

worin $R^1$ und $R^2$ unabhängig voneinander Wasserstoff oder nieder Alkyl und $R^3$ und $R^4$ unabhängig voneinander Wasserstoff, Halogen oder nieder Alkyl bedeuten, mit der Auflage, dass $R^3$ und $R^4$ nicht gleichzeitig Wasserstoff bedeuten,

dadurch gekennzeichnet, dass man

(a) ein 4-Halo-2-phenylchinolin der allgemeinen Formel

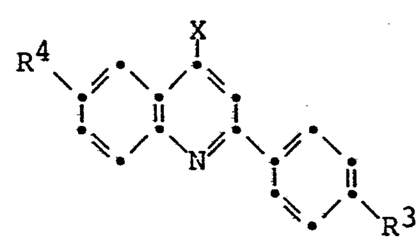

II

worin $R^3$ und $R^4$ obige Bedeutung besitzen und X Halogen bedeutet,

mit einem Isonipecotamid der allgemeinen Formel

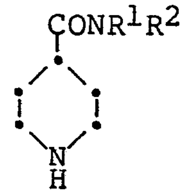

III

worin $R^1$ und $R^2$ obige Bedeutung besitzen, umsetzt, oder (b) eine Verbindung der allgemeinen Formel

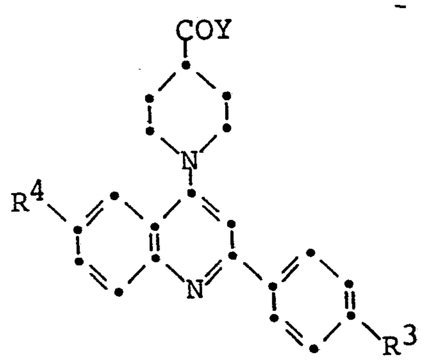

IV

worin $R^3$ und $R^4$ obige Bedeutung besitzen und Y Halogen, Hydroxy oder nieder Alkoxy bedeutet, mit einem Amin der allgemeinen Formel

$$NHR^1R^2 \qquad V$$

worin $R^1$ und $R^2$ obige Bedeutung besitzen, umsetzt.

2. Verfahren gemäss Anspruch 1, worin $R^1$ und $R^2$ unabhängig voneinander Wasserstoff, Methyl oder Aethyl bedeuten.

3. Verfahren gemäss Anspruch 1 oder 2, worin $R^3$ Chlor, Brom,

Methyl oder Aethyl bedeutet.

4. Verfahren gemäss Anspruch 1, dadurch gekennzeichnet, dass man 1-[2-(4-Chlorphenyl)-4-chinolinyl]-N-äthyl-4- piperidincarboxamid herstellt.

5. Verfahren gemäss Anspruch 1, dadurch gekennzeichnet, dass man 1-[2-(4-Chlorphenyl)-6-fluor-4-chinolinyl]-4- piperidincarboxamid herstellt.

6. Verfahren gemäss Anspruch 1, dadurch gekennzeichnet, dass man 1-[2-(4-Chlorphenyl)-4-chinolinyl]-N-methyl-4- piperidincarboxamid herstellt.

7. Verfahren gemäss Anspruch 1, dadurch gekennzeichnet, dass man 1-[2-(4-Chlorphenyl)-4-chinolinyl]-4-piperidincarboxamid herstellt.

0 168 812

Europäisches Patentamt

# EUROPÄISCHER TEILRECHERCHENBERICHT,

der nach Regel 45 des Europäischen Patentübereinkommens für das weitere Verfahren als europäischer Recherchenbericht gilt

EP 85108885.6

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| A | EP - A1 - 0 042 781 (PHARMINDUSTIE)<br>* Formel 1; Anspruch 12 *<br>-- | 1,9,10,<br>12,13 | C 07 D 401/04<br>A 61 K 31/47 |
| A | CHEMICAL ABSTRACTS, Band 97, Nr. 9, 30. August 1982, Columbus, Ohio, USA<br>MOELLER, JOERGEN; PEDERSEN, ERIK B. "Synthesis and mass spectra of isomeric dialkylaminopyridines and dialkylaminoquinolines."<br>Seite 621, Spalte 1, Zusammenfassung-Nr. 72 225c<br>& Chem. Scr. 1982, 19(4), 185-9<br>-- | 1 | |

RECHERCHIERTE SACHGEBIETE (Int. Cl.4)

C 07 D 401/00

## UNVOLLSTÄNDIGE RECHERCHE

Nach Auffassung der Recherchenabteilung entspricht die vorliegende europäische Patentanmeldung den Vorschriften des Europäischen Patentübereinkommens so wenig, daß es nicht möglich ist, auf der Grundlage einiger Patentansprüche sinnvolle Ermittlungen über den Stand der Technik durchzuführen.

Vollständig recherchierte Patentansprüche: 1-13,16

Unvollständig recherchierte Patentansprüche: –

Nicht recherchierte Patentansprüche: 14,15

Grund für die Beschränkung der Recherche:

(Art. 52(4) EPÜ; Verfahren zur therapeutischen Behandlung des meschlichen oder tierischen Körpers)

| Recherchenort | Abschlußdatum der Recherche | Prüfer |
|---|---|---|
| WIEN | 20-09-1985 | HAMMER |

KATEGORIE DER GENANNTEN DOKUMENTEN

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1505.1 03.82

0 168 812

Europäisches Patentamt

# EUROPÄISCHER TEILRECHERCHENBERICHT

EP 85108885.6

| EINSCHLÄGIGE DOKUMENTE | | | |
|---|---|---|---|
| Kategorie | Kennzeichnung des Dokuments mit Angabe, soweit erforderlich, der maßgeblichen Teile | betrifft Anspruch | KLASSIFIKATION DER ANMELDUNG (Int. Cl.4) |
| A | CHEMICAL ABSTRACTS, Band 96, Nr. 7, 15. Februar 1982, Columbus, Ohio, USA<br>HANSEN, BO W.; PEDERSEN, ERIK B. "Phosphoramides. XV. Phosphorus pentoxide amine mixtures as reagents in the synthesis of 2-(dialkylamino)quinolines."<br>Seite 606, Spalte 1, Zusammenfassung-Nr. 52 152c<br>& Liebigs Ann. Chem. 1981, (8), 1485-91<br>-- | 1 | |
| | | | RECHERCHIERTE SACHGEBIETE (Int. Cl.4) |
| A | CHEMICAL ABSTRACTS, Band 94, Nr. 25, 22. Juni 1981, Columbus, Ohio, USA<br>LE FUR, G.; MIZOULE, J.; BURGEVIN, M. C.; FERRIS, O.; HEAULME, M.; GAUTHIER, A.; GUEREMY, C.; UZAN, A. "Multiple benzodiazepine receptors: evidence of a dissociation between anticonflict and anticonvulsant properties by PK 8165 and PK 9084 (two quinoline derivatives)."<br>Seite 33, Spalte 1, Zusammenfassung-Nr. 202 584v<br>& Life Sci. 1981, 28(13), 1439-48<br>---- | 1 | |